# EUROPEAN PATENT SPECIFICATION

(11) **EP 3 715 462 B1**
(45) Date of publication and mention of the grant of the patent: **25.03.2026**
(21) Application number: 17932666.5
(22) Date of filing: 13.12.2017
(51) Int. Cl.: C12N 9/22, C12N 15/67, C12N 15/81

(54) **METHOD FOR REGULATING IN VITRO BIOSYNTHESIS ACTIVITY BY KNOCKING-OUT OF NUCLEASE SYSTEM**
VERFAHREN ZUR REGULIERUNG DER IN-VITRO-BIOSYNTHESE-AKTIVITÄT DURCH NUKLEASESYSTEM-KNOCKOUT
PROCÉDÉ DE RÉGULATION DE L'ACTIVITÉ DE BIOSYNTHÈSE IN VITRO PAR KNOCK-OUT D'UN SYSTÈME DE NUCLÉASE

(30) Priority: 24.11.2017 CN 201711194472
(43) Date of publication of application: 30.09.2020
(73) Proprietor: Kangma-Healthcode (Shanghai) Biotech Co., Ltd, Pudong New Area Shanghai 201321 (CN)
(72) Inventor: GUO, Min, Shanghai 201321 (CN); JIANG, Lingxuan, Shanghai 201321 (CN); ZHENG, Zhuxia, Shanghai 201321 (CN); YU, Xue, Shanghai 201321 (CN)
(74) Representative: Cabinet Chaillot
(86) International application number: PCT/CN2017/115966
(87) International publication number: WO 2019/100456

(56) References cited:
- WO-A2-2006/019876
- CN-A- 106 701 607
- CN-A- 106 978 349
- US-A- 4 668 624
- US-A1- 2016 060 301
- SPIRIN A S: "High-throughput cell-free systems for synthesis of functionally active proteins", TRENDS IN BIOTECHNOLOGY, ELSEVIER PUBLICATIONS, CAMBRIDGE, GB, vol. 22, no. 10, 1 October 2004 (2004-10-01), pages 538 - 545, XP004575232, ISSN: 0167-7799, DOI: 10.1016/J.TIBTECH.2004.08.012
- DATABASE GenBank 25 September 2017 (2017-09-25), DUJON, B. ET AL., Database accession no. XP 456080
- DATABASE GenBank 25 September 2017 (2017-09-25), DUJON, B. ET AL., Database accession no. XM _456080

## Description

### BACKGROUND OF THE INVENTION

### 1. Technical Field

The present invention relates to the field of biotechnology, and more particularly, to an *in vitro* cell-free protein synthesis system and a method for *in vitro* protein synthesis using the system.

### 2. Description of the Related Art

Proteins are important molecules in cells, and involve the execution of almost all functions of cells. The sequence and structure of proteins determine the function thereof. In cells, proteins can act as enzymes to catalyze various biochemical reactions and can act as signaling molecules to coordinate various activities of organisms. Proteins can support biological forms, can store energy, can transport molecules, and can enable organisms to move. In the field of biomedicine, antibodies (a type of protein), as targeted drugs, are an important means to treat cancer and other diseases ^{[1][2]}.

In cells, the regulation of protein translation plays an important role not only in responding to external pressures such as nutritional deficiency, but also in various processes such as cell development and differentiation. Protein translation is divided into four processes, including translation initiation, translation elongation (translation extension), translation termination and ribosome recycling, among which translation initiation is the most regulated process^{[3]}. In the initiation phase of translation, the small subunit (40S) of ribosome binds to (tRNA)ᵢ^{Met}, and recognizes the 5' portion of mRNA with the assistance of translation initiation factors. The small subunit moves downstream and binds to the large subunit (60S) of ribosome at the position of initiation codon (AUG) to form a complete ribosome. Then the translation elongation phase starts^{[4]}.

An *in vitro* biosynthesis system refers to a lysis system based on bacteria, fungi, plant cells or animal cells, which is added with nucleic acid DNA, RNA, substrates and energy source to complete rapid and efficient translation of specific chemical molecules or biomacromolecules (DNA, RNA, and proteins). A commonly used *in vitro* biosynthesis system is an *in vitro* protein synthesis system which uses exogenous mRNA or DNA template and cell lysate to accomplish rapid and efficient translation of exogenous recombinant proteins^{[5]}.

Currently, one of commercially available and commonly used *in vitro* protein synthesis systems is an *in vitro* transcription-translation system (abbreviated as IVTT system), which transcribes DNA template into mRNA intermediate through RNA polymerase, and then completes one-step efficient translation of exogenous proteins by using components including amino acids, ATP, etc. Commonly used commercial *in vitro* protein expression systems include *Escherichia coli* extract (ECE) systems, rabbit reticulocyte lysate (RRL) systems, wheat germ extract (WGE) systems, insect cell extract (ICE) systems, and human source systems.

Nucleic acids of mRNA and DNA are coding substrates in the *in vitro* protein synthesis system, and their stability affects the yield of protein. Nuclease is a kind of protein that hydrolyzes the phosphodiester bond between nucleotides in the first step of nucleic acid degradation. Some nucleases which only act upon RNA are known as ribonuclease (RNase), and some nucleases which only act upon DNA are known as deoxyribonucleases (DNase). According to the action site, nucleases can also be divided into categories of exonuclease and endonuclease. The vast majority of RNAs in cells are degraded through the 5' to 3' exonuclease in the cytoplasm and the 5' to 3' exonuclease in the nucleus, or through protein subunit complex exosome with 3' to 5' exonuclease and/or endonuclease activities in the cytoplasm and nucleus.

CRISPR/Cas (Clustered Regulatory Interspaced Short Palindromic Repeats/CRISPR associated) is a biological defense system which widely exists in bacteria and archaea. Among the CRISPR/Cas systems, a modified type II system (CRISPR/Cas9) has become a widely used tool for genome modification. Under the guidance of guide RNA (gRNA), Cas9 protein recognizes protospacer adjacent motif (PAM) and its upstream 20bp sequence on the genome, and creates a double-stranded nick at the position of the 3^{rd} bp upstream of PAM. In the case where donor DNA is meanwhile provided, the gene cleaved by the CRISPR/Cas9 double-strand can be incorporatively recombined with a new sequence in the manner of HDR to achieve the purpose of gene modification. With respect to *Saccharomyces cerevisiae,* there are lots of examples of genome modification using the CRISPR/Cas9 system, including gene point mutation, gene knock-out, and gene insertion.

There may exist some nucleases in the current *in vitro* synthesis systems. These nucleases, through degrading mRNA and DNA in the *in vitro* synthesis system, may thereby affect the stability of nucleic acids in the *in vitro* synthesis system.

Accordingly, there is an urgent need in the art for developing a method for achieving stable expression of exogenous proteins by stabilizing the nucleic acid.

### SUMMARY OF THE INVENTION

An object of the present invention is to provide a method for achieving stable expression of exogenous proteins by stabilizing nucleic acid.

According to the first aspect, the present invention provides an *in vitro* cell-free protein synthesis system, comprising:
(a) yeast cell extract;
(b) polyethylene glycol;
(c) optional exogenous sucrose; and
(d) optional solvent, wherein the solvent is water or aqueous solvent,

wherein the yeast cell extract is derived from yeast cells, and expression or activity of *EXN53* gene in the yeast cells is reduced through CRISPR-Cas9 gene editing technology compared with that in wild-type yeast cells;
wherein, the content of EXN53 protein in the yeast cell extract is equal to or less than 10%, preferably equal to or less than5%, and more preferably equal to or less than 2%, based on the total weight of the yeast cell extract;
wherein amino acid sequence of the EXN53 protein is SEQ ID NO.:6.

In another preferred embodiment, the content of the EXN53 protein in the yeast cell extract is zero.

In another preferred embodiment, the EXN53 protein is derived from one or more sources of yeasts selected from the group consisting of *Pichia pastoris* and *Kluyveromyces,* preferably derived from *Kluyveromyces.*

In another preferred embodiment, the *Kluyveromyces* comprises *Kluyveromyces marxianus* and/or *Kluyveromyces lactis.*

In another preferred embodiment, the nucleotide sequence of the EXN53 protein is shown as SEQ ID NO.:1.

In another preferred embodiment, the cell-free protein synthesis system further comprises one or more components selected from the group consisting of:
(e1) substrate for synthesizing RNA;
(e2) substrate for synthesizing protein;
(e3) magnesium ion;
(e4) potassium ion;
(e5) buffer;
(e6) RNA polymerase; and
(e7) energy regeneration system.

In another preferred embodiment, the cell-free protein synthesis system further comprises one or more components selected from the group consisting of:
(e8) heme; and
(e9) spermidine.

In another preferred embodiment, the yeast cells are derived from one or more sources of yeasts selected from the group consisting of: *Pichia pastoris, Kluyveromyces,* and a combination thereof; preferably, the yeast cells comprise *Kluyveromyces* cells, and more preferably *Kluyveromyces marxianus* cells and/or *Kluyveromyces lactis* cells.

In another preferred embodiment, the yeast cell extract is an aqueous extract of yeast cells.

In another preferred embodiment, the yeast cell extract does not contain yeast endogenous long-chain nucleic acid molecules.

In another preferred embodiment, the yeast cell extract is prepared by using a method comprising the following steps:
(i) providing yeast cells;
(ii) washing the yeast cells to obtain washed yeast cells;
(iii) treating the washed yeast cells with a cell lysis treatment to obtain a crude yeast extract; and
(iv) treating the crude yeast extract via solid-liquid separation to obtain the liquid phase, i.e., the yeast cell extract.

In another preferred embodiment, the solid-liquid separation includes centrifugation.

In another preferred embodiment, the centrifugation is carried out in a liquid state.

In another preferred embodiment, the centrifugation condition is in a range of 5,000×g -100,000×g, more preferably in a range of 8,000×g -30,000×g.

In another preferred embodiment, the centrifugation time is in a range from 0.5 hour to 2 hours, and more preferably, from 20 minutes to 50 minutes.

In another preferred embodiment, the centrifugation is carried out at 1-10 °C, and more preferably, at 2- 6 °C.

In another preferred embodiment, the washing treatment is carried out using a washing solution of pH 7-8 (preferably pH 7.4).

In another preferred embodiment, the washing solution is selected from the group consisting of potassium 4-hydroxyethylpiperazine ethanesulfonate, potassium acetate, magnesium acetate, and combinations thereof.

In another preferred embodiment, methods for cell lysis treatment comprise high-pressure lysis, freeze-thaw (e.g., treatment at liquid-nitrogen low temperature) lysis, etc.

In another preferred embodiment, the substrate for synthesizing RNA includes: nucleoside monophosphates, nucleoside triphosphates, and a combination thereof.

In another preferred embodiment, the substrate for synthesizing protein includes: 1 to 20 kinds of natural amino acids, and unnatural amino acids.

In another preferred embodiment, the magnesium ion comes from a magnesium ion source, and the magnesium ion source is magnesium acetate, magnesium glutamate, or a combination thereof.

In another preferred embodiment, the potassium ion comes from a potassium ion source, and the potassium ion source is potassium acetate, potassium glutamate, or a combination thereof.

In another preferred embodiment, the energy regeneration system is selected from the group consisting of a phosphocreatine/phosphocreatinase system, glycolysis pathway and its intermediate product energy systems, and combinations thereof.

In another preferred embodiment, the cell-free protein synthesis system further comprises (f1) synthetic tRNA.

In another preferred embodiment, the buffer is selected from the group consisting of 4-hydroxyethyl piperazineethanesulfonic acid, tris(hydroxymethyl)aminomethane, and a combination thereof.

In another preferred embodiment, the cell-free protein synthesis system further comprises (g1) an exogenous DNA molecule for guiding protein synthesis.

In another preferred embodiment, the DNA molecule is linear.

In another preferred embodiment, the DNA molecule is circular.

In another preferred embodiment, the DNA molecule contains a sequence encoding an exogenous protein.

In another preferred embodiment, the sequence encoding the exogenous protein includes a genomic sequence and a cDNA sequence.

In another preferred embodiment, the sequence encoding the exogenous protein further comprises a promoter sequence, a 5' untranslated sequence, a 3' untranslated sequence, or a combination thereof.

In another preferred embodiment, the cell-free protein synthesis system comprises components selected from the group consisting of: 4-hydroxyethyl piperazine ethanesulfonic acid, potassium acetate, magnesium acetate, nucleoside triphosphates, amino acids, creatine phosphate (phosphocreatine), dithiothreitol (DTT), creatine phosphokinase (phosphocreatinase), RNA polymerase, and combinations thereof.

In another preferred embodiment, the polyethylene glycol is selected from the group consisting of: PEG3000, PEG8000, PEG6000, PEG3350, and combinations thereof.

In another preferred embodiment, the polyethylene glycol includes polyethylene glycol with a molecular weight of 200-10,000 Da, preferably, polyethylene glycol with a molecular weight of 3,000-10,000 Da.

In another preferred embodiment, in the protein synthesis system, the concentration (v/v) of the component (a) is in a range from 20% to 70%, preferably from 30% to 60%, more preferably from 40% to 50%, based on the total volume of the protein synthesis system.

In another preferred embodiment, in the protein synthesis system, the concentration (w/v, for example, g/mL) of the component (b) is in a range from 0.1% to 8%, preferably from 0.5% to 4%, and more preferably from 1% to 2%.

In another preferred embodiment, in the protein synthesis system, the concentration of the component (c) is in a range from 0.2% to 4%, preferably from 0.5% to 4%, and more preferably from 0.5% to 1%, based on the total volume of the protein synthesis system.

In another preferred embodiment, the nucleoside triphosphates are selected from the group consisting of adenosine triphosphate (ATP), guanosine triphosphate (GTP), cytidine triphosphate (CTP), uridine triphosphate (UTP), and combinations thereof.

In another preferred embodiment, in the protein synthesis system, the concentration of the component (e1) is in a range from 0.1 mM to 5 mM, preferably from 0.5 mM to 3 mM, and more preferably from 1 mM to 1.5 mM.

In another preferred embodiment, the amino acids are selected from the group consisting of glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, histidine, and combinations thereof.

In another preferred embodiment, the amino acids include amino acids of D-type and/or amino acids of L-type.

In another preferred embodiment, in the protein synthesis system, the concentration of the component (e2) is in a range from 0.01 mM to 0.48 mM, preferably from 0.04 mM to 0.24 mM, more preferably from 0.04 mM to 0.2 mM, and most preferably 0.08 mM.

In another preferred embodiment, in the protein synthesis system, the concentration of the component (e3) is in a range from 1 mM to 10 mM, preferably from 1 mM to 5 mM, and more preferably from 2 mM to 4 mM.

In another preferred embodiment, in the protein synthesis system, the concentration of the component (e4) is in a range from 30 mM to 210 mM, preferably from 30 mM to 150 mM, and more preferably from 30 mM to 60 mM.

In another preferred embodiment, in the protein synthesis system, the concentration of the component (e6) is in a range from 0.01 mg/mL to 0.3 mg/mL, preferably from 0.02 mg/mL to 0.1 mg/mL, and more preferably from 0.027 mg/mL to 0.054 mg/mL.

In another preferred embodiment, in the protein synthesis system, the concentration of 4-hydroxyethyl piperazineethanesulfonic acid is in a range from 5 mM to 50 mM, preferably from 10 mM to 50 mM, more preferably from 15 mM to 30 mM, and more preferably from 20 mM to 25 mM.

In another preferred embodiment, in the protein synthesis system, the concentration of potassium acetate is in a range from 20 mM to 210 mM, preferably from 30 mM to 210 mM, more preferably from 30 mM to 150 mM, and more preferable from 30 mM to 60 mM.

In another preferred embodiment, in the protein synthesis system, the concentration of magnesium acetate is in a range from 1 mM to 10 mM, preferably from 1 mM to 5 mM, and more preferably from 2 mM to 4 mM.

In another preferred embodiment, in the protein synthesis system, the concentration of phosphocreatine is in a range from 10 mM to 50 mM, preferably from 20 mM to 30 mM, and more preferably 25 mM.

In another preferred embodiment, in the protein synthesis system, the concentration of heme is in a range from 0.01 mM to 0.1 mM, preferably from 0.02 mM to 0.08 mM, more preferably from 0.03 mM to 0.05 mM, and most preferably 0.04 mM.

In another preferred embodiment, in the protein synthesis system, the concentration of spermidine is in a range from 0.05 mM to 1 mM, preferably from 0.1 mM to 0.8 mM, more preferably from 0.2 mM to 0.5 mM, more preferably from 0.3 mM to 0.4 mM, and most preferably 0.04 mM.

In another preferred embodiment, in the protein synthesis system, the concentration of dithiothreitol (DTT) is in a range from 0.2 mM to 15 mM, preferably from 0.2 mM to 7 mM, and more preferably from 1 mM to 2 mM.

In another preferred embodiment, in the protein synthesis system, the concentration of creatine phosphokinase is in a range from 0.1 mg/mL to 1 mg/mL, preferably from 0.2 mg/mL to 0.5 mg/mL, and more preferably 0.27 mg/mL.

In another preferred embodiment, the RNA polymerase is T7 RNA polymerase.

In another preferred embodiment, in the protein synthesis system, the concentration of T7 RNA polymerase is in a range from 0.01 mg/mL to 0.3 mg/mL, preferably from 0.02 mg/mL to 0.1 mg/mL, and more preferably from 0.027 mg/mL to 0.054 mg/mL.

In another preferred embodiment, the cell-free *in vitro* synthesis system has the following properties:
in the synthesis system, the total amount of synthesized proteins reaches 3 µg of protein per milliliter (mL) of the synthesis system.

In another preferred embodiment, the cell-free protein synthesis system comprises the following components:

| | General range | Preferred range |
|---|---|---|
| 4-hydroxyethyl piperazine ethanesulfonic acid | 5-40 mM | 10-30 mM (Preferably, 20-30 mM) |
| magnesium acetate | 1-10 mM | 2-5 mM |
| potassium acetate | 20-150 mM | 30-75 mM |
| DTT | 0.5-5 mM | 1-2 mM |
| phosphocreatine | 15-50 mM | 20-30 mM |
| creatine phosphokinase | 0.1-0.5 mg/mL | 0.2-0.3 mg/mL |
| 4 kinds of ribonucleotides | Respectively 0.5-5 mM | Respectively 1.0-2.0 mM |
| DNA template | 2-50 ng/µL | 5-25 ng/µL |
| RNA polymerase | 0.01-0.3 mg/mL | 0.02-0.10 mg/mL |
| PEG | 0.5-5%(w/v) | 1-3%(w/v) |

In another preferred embodiment, the cell-free protein synthesis system also comprises the following components:

| | General range | Preferred range |
|---|---|---|
| spermidine | 0.2-0.4 mM | 0.3-0.4 mM |
| heme | 0.01-0.04 mM | 0.03-0.04mM |

In another preferred embodiment, the PEG is selected from the group consisting of PEG3350, PEG3000, PEG8000, and combinations thereof.

According to the third aspect, the present invention provides a method for producing the *in vitro* cell-free protein synthesis system according to the first aspect of the present invention, wherein, the method comprises a step of:
mixing the components of (a) the yeast cell extract, (b) polyethylene glycol, (c) optional exogenous sucrose, and (d) optional solvent, to obtain the *in vitro* cell-free protein synthesis system according to the first aspect of the present invention, wherein, the solvent is water or aqueous solvent; the yeast cell extract comprises EXN53 protein, and the content of the EXN53 protein in the yeast cell extract is equal to or less than 10%, preferably equal to or less than 5%, and more preferably equal to or less than 2%.

In another preferred embodiment, in the protein synthesis system, the concentration (v/v) of the component (a) is in a range from 20% to 70%, preferably from 30% to 60%, and more preferably from 40% to 50%, based on the total volume of the protein synthesis system.

In another preferred embodiment, in the protein synthesis system, the concentration (w/v, for example, g/mL) of the component (b) is in a range from 0.1% to 8%, preferably from 0.5% to 4%, and more preferably from 1% to 2%.

According to the fourth aspect of the present invention provides a method for *in vitro* synthesis of protein, wherein, the method comprises steps of:
(i) providing the *in vitro* cell-free protein synthesis system according to the first aspect of the present invention, and adding exogenous DNA molecules for guiding protein synthesis, wherein in the protein synthesis system, the content of EXN53 protein is equal to or less than 10%, preferably equal to or less than5%, and more preferably equal to or less than 2%; and
(ii) incubating the protein synthesis system provided in the step (i) for a period of time T1 under suitable conditions to synthesize the protein encoded by the exogenous DNA.

In another preferred embodiment, the method further comprises: (iii) optionally, isolating or detecting the protein encoded by the exogenous DNA from the protein synthesis system.

In another preferred embodiment, the exogenous DNA is derived from a prokaryote or a eukaryote.

In another preferred embodiment, the exogenous DNA is derived from animal, plant or pathogen.

In another preferred embodiment, the exogenous DNA is derived from mammal, preferably primate or rodent, including human, mouse and rat.

In another preferred embodiment, the coding sequence of the exogenous protein encodes an exogenous protein selected from the group consisting of: luciferin, luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, the exogenous protein is selected from the group consisting of: luciferin, luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, the exogenous DNA encodes an exogenous protein which is selected from the group consisting of: luciferin, luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, the protein encoded by the exogenous DNA is selected from the group consisting of: luciferin, luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In another preferred embodiment, in the step (ii), the reaction temperature is in a range from 20 °C to 37 °C, preferably from 20 °C to 25 °C.

In another preferred embodiment, in step (ii), the reaction time is in a range from 1 to 6 hours, preferably from 2 to 4 hours.

According to the fifth aspect, the present invention provides an engineering strain, wherein the engineering strain is a *Kluyveromyces* strain, and the expression level or activity of *EXN53* gene (a nuclease gene) in the engineering strain is reduced through CRISPR-Cas9 gene editing technology compared with that in wild-type *Kluyveromyces* strain; wherein, the EXN53 gene is a nuclease gene;
wherein amino acid sequence of the EXN53 protein is SEQ ID NO.:6.

In another preferred embodiment, the term "reduced" means that the expression level of the *EXN53* gene is equal to or less than 10%, preferably equal to or less than 5%, and more preferably equal to or less than 2%.

In another preferred embodiment, the term "reduced" means that the reduction of the expression level or activity of the *EXN53* gene meets the following condition:
ratio of A1 to A0 is equal to or less than 30%, preferably equal to or less than 10%, more preferably equal to or less than 5%, more preferably equal to or less than 2%, and most preferably in a range of 0 to 2%;
wherein, A1 corresponds to the expression level of the *EXN53* gene, and A0 corresponds to the expression level of wild-type *EXN53* gene; or
A1 corresponds to the activity of the *EXN53* gene, and A0 corresponds to the activity of the wild-type *EXN53* gene.

In another preferred embodiment, the expression level or activity of the *EXN53* gene (a nuclease gene) in the strain is reduced by using a method selected from the group consisting of: gene mutation, gene knock-out, gene disruption, RNA interference technique, Crispr technique, and combinations thereof.

According to the sixth aspect, the present invention provides a use of the engineering strain according to the fifth aspect of the present invention, for improving the efficiency of *in vitro* protein synthesis.

It should be understood that, within the scope of the present invention, the above-mentioned technical features and the technical features specifically described hereinafter (e.g. embodiments or examples) can be combined with each other, whereby forming new or preferred technical solutions. For the simplicity, details will not be repeated herein.

### BRIEF DESCRIPTION OF THE DRAWINGS

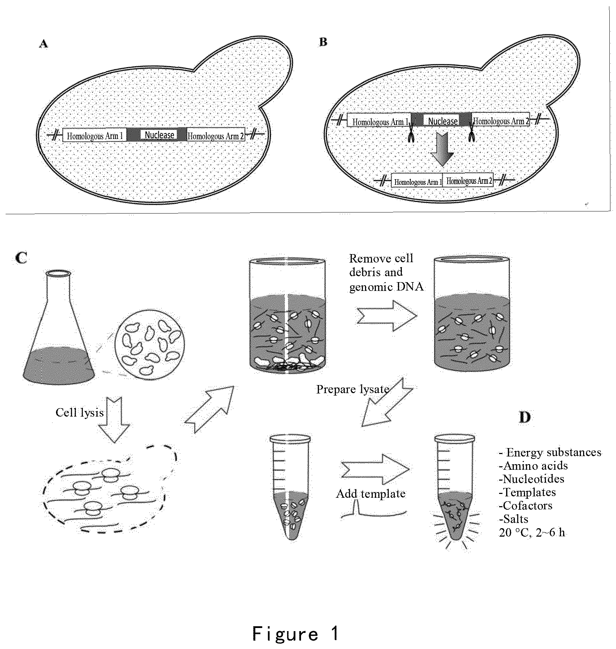

Figure 1 shows a design route for improving the stability of nucleic acid by downregulating the nuclease in an *in vitro* biosynthesis system, via performing analysis on and making specific modification to the nuclease gene in the *K. lactis* genome. (A) *K. lactis* wild-type cells. (B) *K. lactis* cells in which the nuclease has been modified. (C) Collect lysate of *K. lactis* cells in which the nuclease has been modified. (D) A yeast cell solution is prepared from yeast strain in which specific genes have been modified, wherein the solution has a characteristic of more stable nucleic acid. The yeast cell solution is used for preparing an enhanced *in vitro* biosynthesis system.

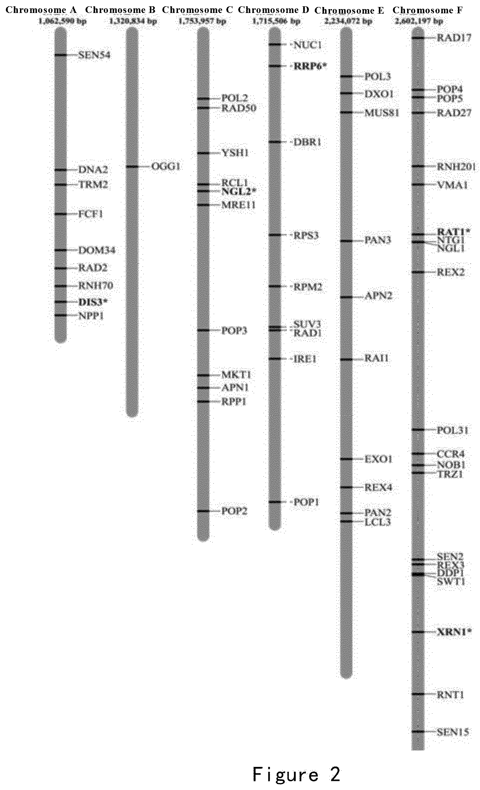

Figure 2 shows the distribution and functional analysis of all 61 nuclease genes in the *K. lactis* genome. A-F correspond to six chromosomes of *K. lactis;* wherein, nuclease genes located at the A chromosome are: *KLSEN54, KLDNA2, KLTRM2, KLFCF1, KLDOM34, KLRAD2, KLRNH70, KLDIS3* and *KLNPP1;* nuclease genes located at the B chromosome is: *KLOGG1;* nuclease genes located at the C chromosome are: *KLPOL2, KLRAD50, KLYSH1, KLRCL1, KLNGL2, KLMRE11, KLPOP3, KLMKT1, KLAPN1, KLRPP1* and *KLPOP2;* nuclease genes located at the D chromosome are: *KLNUC1, KLRRP6, KLDBR1, KLRPS3, KLRPM2, KLSUV3, KLRAD1, KLIRE1* and *KLPOP1;* nuclease genes located at the E chromosome are: *KLPOL3, KLDXO1, KLMUS81, KLPAN3, KLAPN2, KLRAI1, KLEXO1, KLREX4, KLPAN2* and *KLLCL3;* nuclease genes located at the F chromosome are: *KLRAD17, KLPOP4, KLPOP5, KLRAD27, KLRNH201, KLVMA1, KLRAT1, KLNGL1, KLREX2, KLPOL31, KLCCR4, KLTRZ1, KLSEN2, KLREX3, KLSWT1, KLEXN53, KLRNT1, KLSEN15, KLNTG1, KLNOB1* and *KLDDP1.*

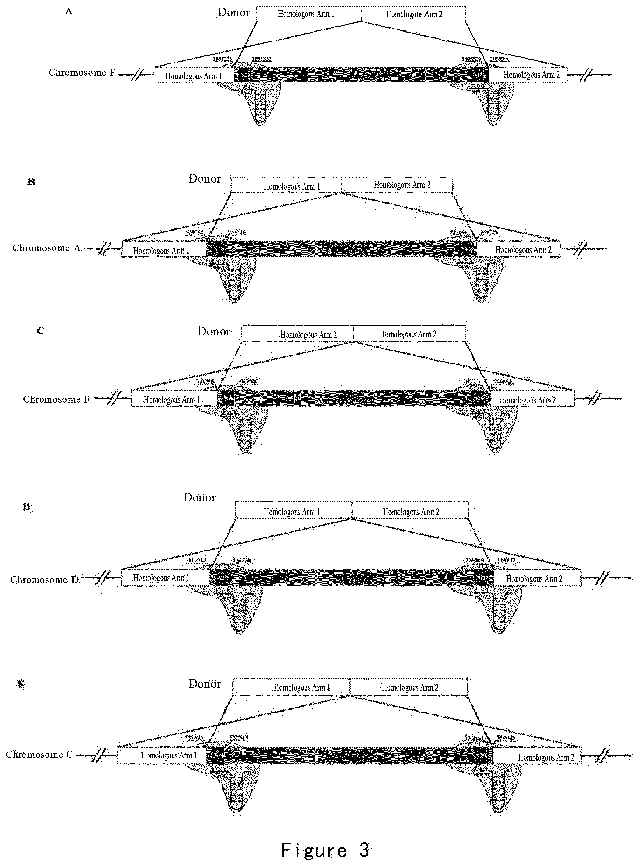

Figure 3 shows the sequences of five nuclease genes in the *K. lactis* genome which have been genetically modified and corresponding CRISPR system design routes. Two homologous arms of a donor DNA, homologous arm 1 and homologous arm 2, are gene sequences located at about 1,000 bp upstream and downstream of the ORF of the nuclease gene to be knocked out, respectively; constructing two gRNAs which respectively recognize PAM sequences at the 5' terminal and 3' terminal of the ORF of the nuclease gene to be knocked out at the same time and guide Cas9 nuclease to cleave DNA in a site-specific manner, then homologous recombination happens after DSB (double-strand break) occurs, thereby achieving gene knock-out.

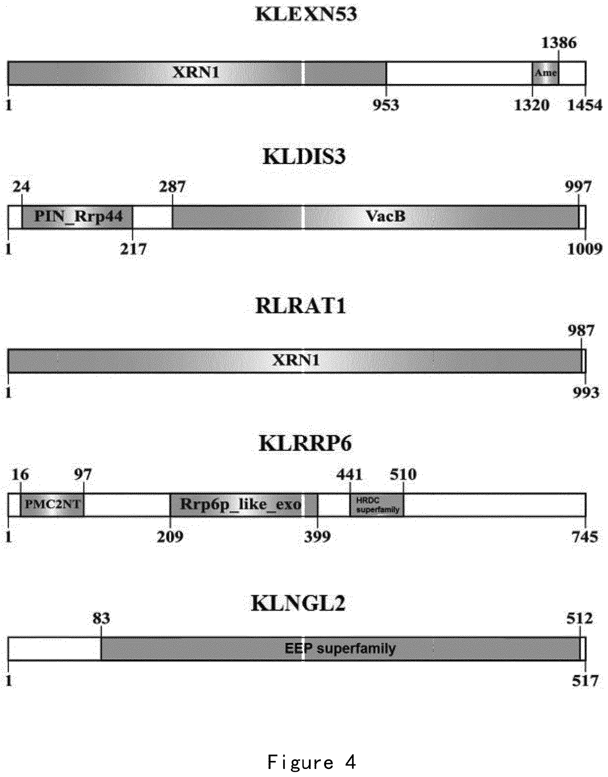

Figure 4 shows a schematic diagram of five nuclease protein domains in the *K. lactis* genome.

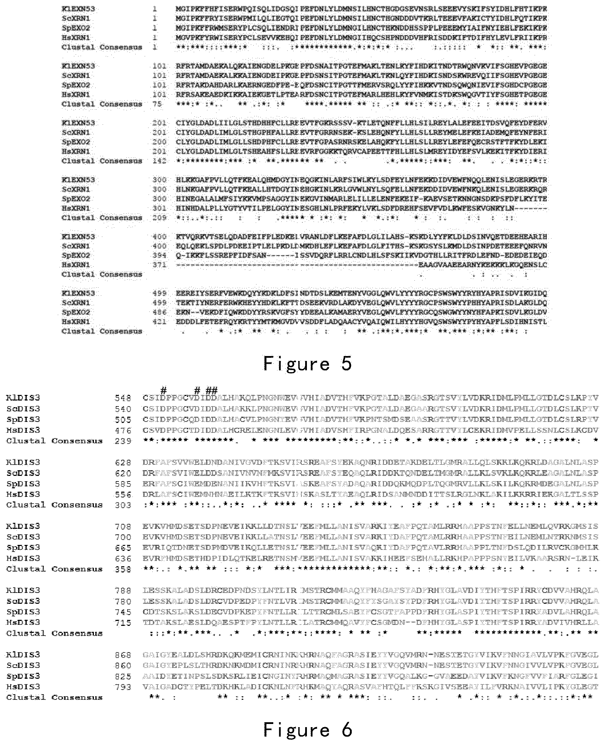

Figure 5 shows comparisons of parts of amino acid sequences between the KLEXN53 protein and the homologous protein ScEXN53 (KEGG No.: YGL173C) in *Saccharomyces cerevisiae,* between the KLEXN53 protein and the homologous protein SpEXO2 (KEGG No.: SPAC17A5.14) in *Schizosaccharomyces pombe,* as well as between the KLEXN53 protein and the homologous protein HsEXN53 (KEGG No.:54464) in *Homo sapiens,* respectively, wherein active sites are marked with #.
Figure 6 shows comparisons of parts of amino acid sequences between the K1DIS3 protein and the homologous protein ScDIS3 (KEGG No.: YOL021C) in *Saccharomyces cerevisiae,* between the K1DIS3 protein and the homologous protein SpDIS3 (KEGG No.:SPBC26H8.10) in *Schizosaccharomyces pombe,* as well as between the K1DIS3 protein and the homologous protein HsDIS3 (KEGG No.: 22894) in *Homo sapiens,* respectively, wherein active sites are marked with #.

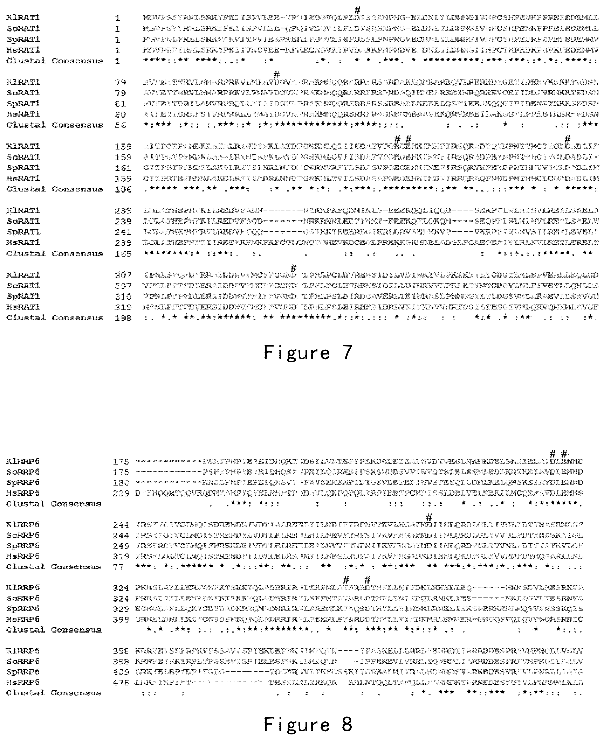

Figure 7 shows comparisons of parts of amino acid sequences between the KLRAT1 protein and the homologous protein ScRAT1 (KEGG No.: YOR048C) in *Saccharomyces cerevisiae,* between the KLRAT1 protein and the homologous protein SpRAT1 (KEGG No.: SPAC26A3.12c) in *Schizosaccharomyces pombe,* as well as between the KLRAT1 protein and the homologous protein HsRAT1 (KEGG No.: 22803) in *Homo sapiens,* respectively, wherein active sites are marked with #.
Figure 8 shows comparisons of parts of amino acid sequences between the KLRRP6 protein and the homologous protein ScRRP6 (KEGG No.: YOROO1W) in *Saccharomyces cerevisiae,* between the KLRRP6 protein and the homologous protein SpRRP6 (KEGG No.: SPAC1F3.01) in *Schizosaccharomyces pombe,* as well as between the KLRRP6 protein and the homologous protein HsRRP6 (KEGG No.: 5394) in *Homo sapiens,* respectively, wherein active sites are marked with #.

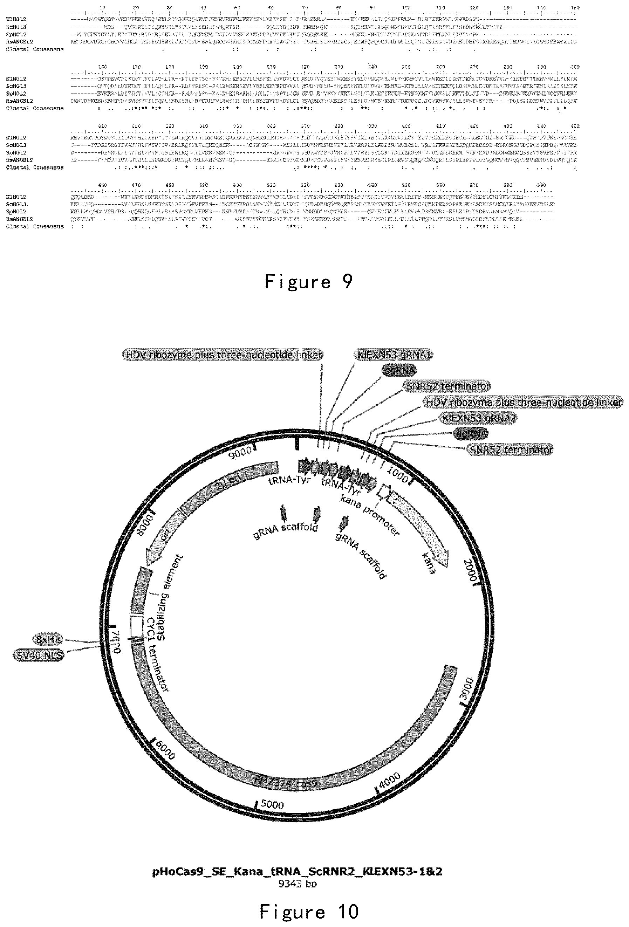

Figure 9 shows comparisons of parts of amino acid sequences between the KLNGL2 protein and the homologous protein ScNGL3 (KEGG No.: YML118W) in *Saccharomyces cerevisiae,* between the KLNGL2 protein and the homologous protein SpNGL2 (KEGG No.: SPBC9B6.11c) in *Schizosaccharomyces pombe,* as well as between the KLNGL2 protein and the homologous protein HsANGEL2 (KEGG No.: 90806) in *Homo sapiens,* respectively.
Figure 10 shows a plasmid profile of pHoCas9_SE_Kana_tRNA_ScRNR2_KLEXN53-1&2. gRNA1 and gRNA2 of *KLEXN53* are two gRNAs at the 5'-terminal and 3'-terminal of the ORF of the *KLEXN53* gene, respectively, with tRNA-Tyr promoter and SNR52 terminator. PMZ374-cas9 is an optimized cas9. The plasmid has a kana selection marker.

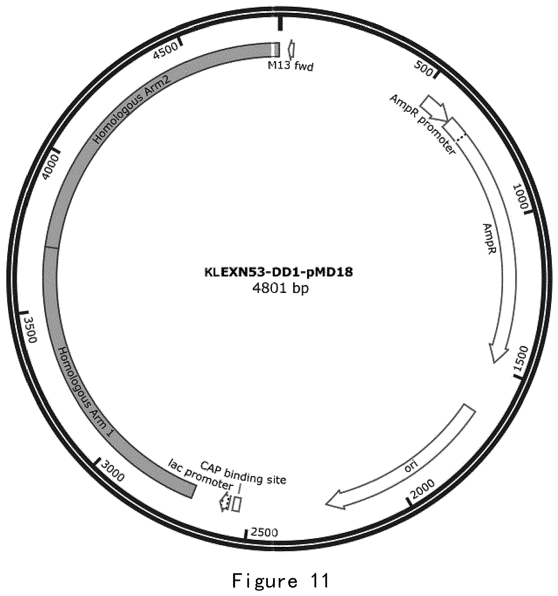

Figure 11 shows a plasmid profile of KLEXN53-DD1-pMD18. Homologous arm 1 and homologous arm 2 are the gene sequences at about 1000 bp upstream and downstream of the ORF of the *KLEXN53* gene, respectively. The plasmid has an Amp selection marker.

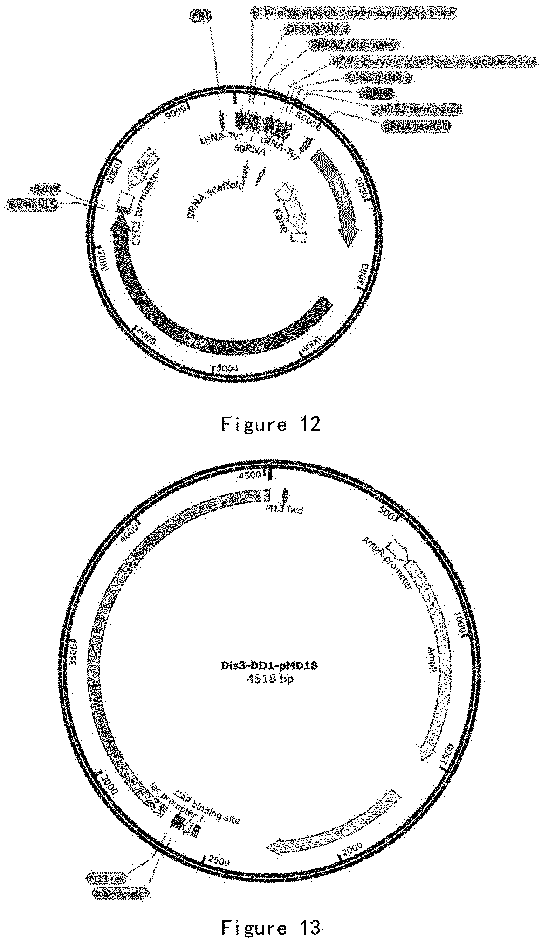

Figure 12 shows a plasmid profile of pHoCas9_SE_Kana_tRNA_ScRNR2_KLDIS3-1&2. gRNA1 and gRNA2 of *KLDis3* are two gRNAs at the 5'-terminal and 3'-terminal of the ORF of the *KLDis3* gene, respectively, with tRNA-Tyr promoter and SNR52 terminator. PMZ374-cas9 is an optimized cas9. The plasmid has a kana selection marker.
Figure 13 shows a plasmid profile of KLDIS3-DD1-pMD18. Homologous arm 1 and homologous arm 2 are the gene sequences at about 1000 bp upstream and downstream of the ORF of the *KLDis3* gene, respectively. The plasmid has an Amp selection marker.

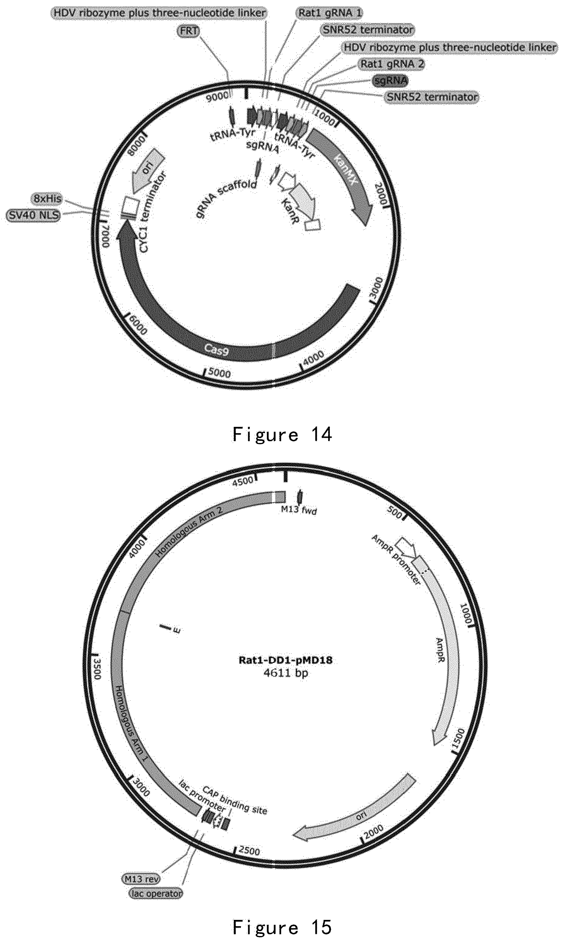

Figure 14 shows a plasmid profile of pHoCas9_SE_Kana_tRNA_ScRNR2_KLRat1-1&2. gRNA1 and gRNA2 of *KLRat1* are two gRNAs at the 5'-terminal and 3'-terminal of the ORF of the *KLRat1* gene, respectively, with tRNA-Tyr promoter and SNR52 terminator. PMZ374-cas9 is an optimized cas9. The plasmid has a kana selection marker.
Figure 15 shows a plasmid profile of KLRat1-DD1-pMD18. Homologous arm 1 and homologous arm 2 are the gene sequences at about 1000 bp upstream and downstream of the ORF of the *KLRat1* gene, respectively. The plasmid has an Amp selection marker.

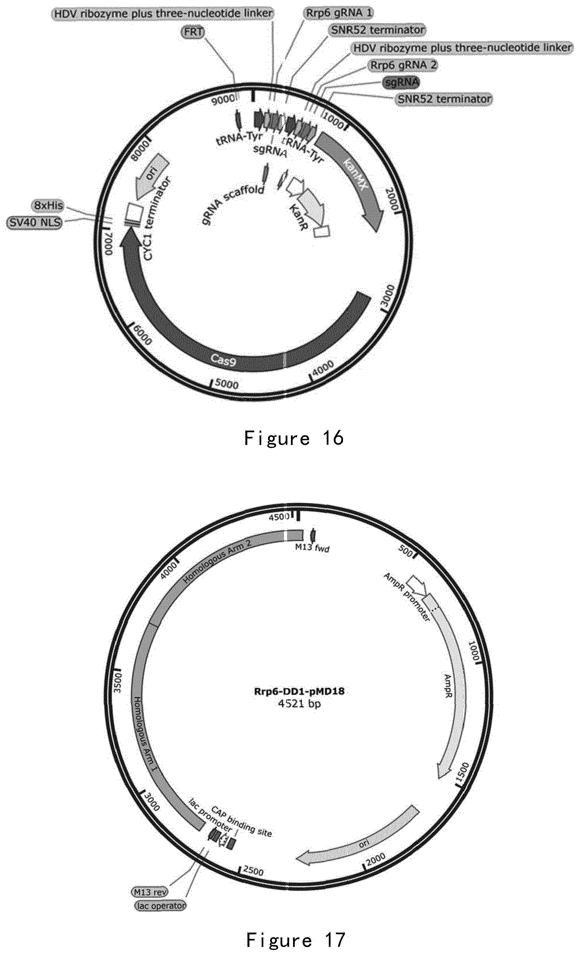

Figure 16 shows a plasmid profile of pHoCas9_SE_Kana_tRNA_ScRNR2_KLRrp6-1&2. gRNA1 and gRNA2 of *KLRrp6* are two gRNAs at the 5'-terminal and 3'-terminal of the ORF of the *KLRrp6* gene, respectively, with tRNA-Tyr promoter and SNR52 terminator. PMZ374-cas9 is an optimized cas9. The plasmid has a kana selection marker.
Figure 17 shows a plasmid profile of KLRrp6-DD1-pMD18. Homologous arm 1 and homologous arm 2 are the gene sequences at about 1000 bp upstream and downstream of the ORF of the *KLRrp6* gene, respectively. The plasmid has an Amp selection marker.

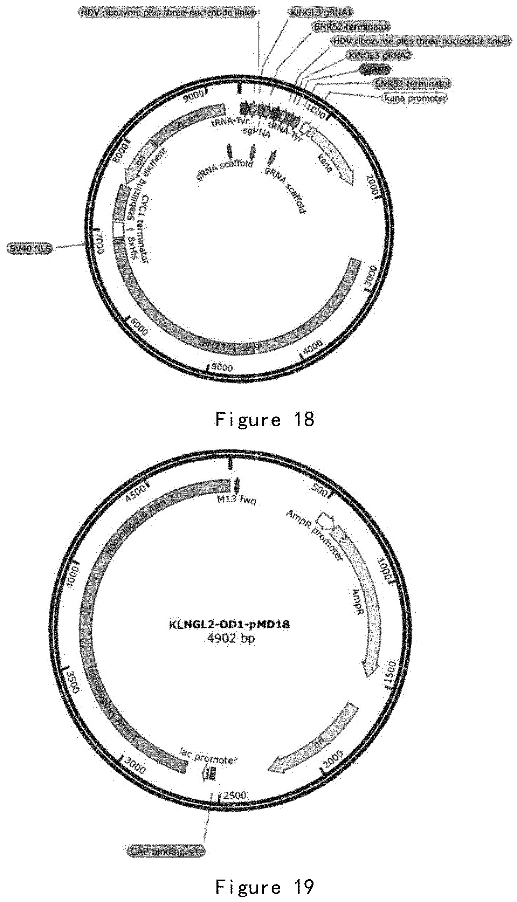

Figure 18 shows a plasmid profile of pHoCas9_SE_kana_tRNA_ScRNR2_KLNGL2-1&2. gRNA1 and gRNA2 of KLNGL3 are two gRNAs at the 5'-terminal and 3'-terminal of the ORF of the *KLNGL3* gene, respectively, with tRNA-Tyr promoter and SNR52 terminator. PMZ374-cas9 is an optimized cas9. The plasmid has a kana selection marker.
Figure 19 shows a plasmid profile of KLNGL2-DD1-pMD18. Homologous arm 1 and homologous arm 2 are the gene sequences at about 1000 bp upstream and downstream of the ORF of the *KLNGL3* gene, respectively. The plasmid has an Amp selection marker.

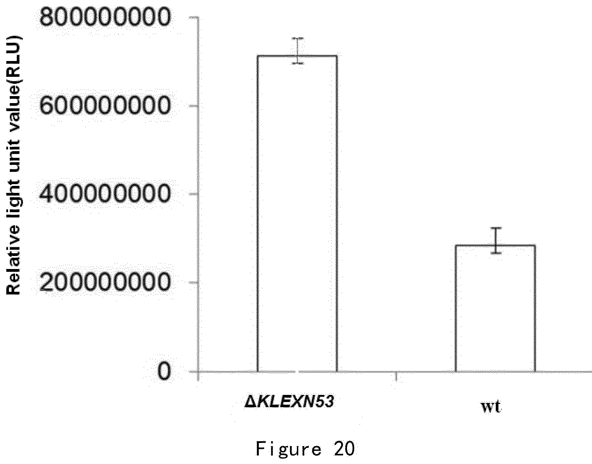

Figure 20 shows the graph of *in vitro* translation activity assay data of the modified strain. The fluorescence intensity of firefly fluorescent protein (Fluc) is used to indicate the synthesis ability of the recombinant protein in the *in vitro* biosynthesis system. Wherein, wt represents wild-type *Kluyveromyces,* Δ*KLEXN53* represents a *Kluyveromyces* strain with *KLEXN53* gene having been knocked-out.

### DETAILED DESCRIPTION

The invention is set out in the appended set of claims. After extensive and in-depth research, five nucleases were selected from many nucleases through extensive screening and groping. Surprisingly, it was found that the down-regulation or knock-out of EXN53, one of the nucleases, can improve the stability of the nucleic acid, and greatly increase the efficiency of protein production *in vitro* protein synthesis system. Specifically, after the down-regulation or knock-out of EXN53, the luciferase activity of Δ*KLEXN53* yeast strain in the IVTT system was twice or more folds of that of wild-type strain. On this basis, inventors have completed the present invention.

Unless otherwise stated, all % used in the present invention refer to percentage concentration by weight (wt%).

### YEAST-BASED IN VITRO PROTEIN SYNTHESIS SYSTEM

Yeast has advantages of simple culture, efficient protein folding and post-translational modification. Among yeasts, *Saccharomyces cerevisiae* and *Pichia pastoris* are model organisms for expressing complex eukaryotic proteins and membrane proteins. In addition, yeast can also be used as a raw material for the preparation of an *in vitro* translation system.

*Kluyveromyces* is an ascosporogenous yeast. Among *Kluyveromyces, Kluyveromyces marxianus* and *Kluyveromyces lactis* are yeasts widely used in industry. Compared with other yeasts, *Kluyveromyces lactis* has many advantages, such as super secretion ability, better large-scale fermentation characteristics, conforming to food safety level, having the ability of post-translational modification of proteins, etc.

In the present invention, the yeast-based *in vitro* protein synthesis system is not particularly limited. A preferred yeast-based *in vitro* protein synthesis system is a *Kluyveromyces-based* expression system (more preferably, a *Kluyveromyces lactis* based expression system).

In the present invention, the yeast-based *in vitro* protein synthesis system comprises:
(a) yeast cell extract;
(b) polyethylene glycol;
(c) optional exogenous sucrose; and
(d) optional solvent, and the solvent is water or aqueous solvent;

wherein the yeast cell extract is derived from yeast cells, and expression or activity of *EXN53* gene in the yeast cells is reduced through CRISPR-Cas9 gene editing technology compared with that in wild-type yeast cells;
wherein, and the yeast cell extract comprises EXN53 protein, and the content of the EXN53 protein in the yeast cell extract is equal to or less than 10%, preferably equal to or less than 5%, and more preferably equal to or less than 2%, based on the total weight of the yeast cell extract;
wherein amino acid sequence of the EXN53 protein is SEQ ID NO.:6.

In a preferred embodiment, the EXN53 protein is derived from one or more sources of yeasts selected from the group consisting of *Pichia pastoris* and *Kluyveromyces,* preferably derived from *Kluyveromyces* (e.g., *Kluyveromyces marxianus, Kluyveromyces lactis*).

In the present invention, *Kluyveromyces* (such as *Kluyveromyces lactis*) is not particularly limited, and includes any *Kluyveromyces* (such as *Kluyveromyces lactis*) strain that can improve the efficiency of synthesizing proteins.

In a preferred embodiment, the protein sequence of the EXN53 protein is shown as SEQ ID NO.:6, and the nucleotide sequence of the *EXN53* is shown as SEQ ID NO.:1.

In a particularly preferred embodiment, the *in vitro* protein synthesis system provided in the present invention comprises: yeast cell extract, 4-hydroxyethyl piperazineethanesulfonic acid, potassium acetate, magnesium acetate, adenosine triphosphate (ATP), guanosine triphosphate (GTP), cytidine triphosphate (CTP), uridine triphosphate (UTP), amino acid mixture, phosphocreatine, dithiothreitol (DTT), creatine phosphokinase, RNase inhibitor, luciferin, DNA of luciferase, and RNA polymerase.

In the present invention, the RNA polymerase is not particularly limited. The RNA polymerase can be one or more RNA polymerases, and a typical RNA polymerase is T7 RNA polymerase.

In the present invention, the proportion of the yeast cell extract in the *in vitro* protein synthesis system is not particularly limited. Generally, the yeast cell extract in the *in vitro* protein synthesis system accounts for 20% to 70%, preferably, 30% to 60%, more preferably, 40% to 50%, by volume.

In the present invention, the yeast cell extract does not contain intact cells, and a typical yeast cell extract comprises: ribosome, transfer RNA, aminoacyl tRNA synthetase, and factors required for protein translation including initiation factors, elongation factors and termination release factors (release factors mediating termination). Furthermore, the yeast extract also comprises some other proteins derived from the cytoplasm of yeast cells, especially soluble proteins.

In the present invention, the protein content of the yeast cell extract is 20-100 mg/mL, preferably 50-100 mg/mL. The method for measuring the protein content is Coomassie brilliant blue assay.

In the present invention, the method for preparing the yeast cell extract is not particularly limited. A preferred preparation method comprises the following steps:
(i) providing yeast cells;
(ii) washing the yeast cells to obtain washed yeast cells;
(iii) treating the washed yeast cells with a cell lysis treatment to obtain a crude yeast extract; and
(iv) treating the crude eukaryotic extract via solid-liquid separation to obtain the liquid phase, that is the yeast cell extract.

In a preferred embodiment, the method of solid-liquid separation is not particularly limited. A preferred method is centrifugation.

In a preferred embodiment, the centrifugation is carried out in a liquid state.

In the present invention, the centrifugation condition is not particularly limited. A preferred centrifugation condition is in a range of 5,000×g -100,000×g, more preferably in a range of 8,000×g -30,000×g.

In the present invention, the centrifugation time is not particularly limited. A preferred centrifugation time is in the range from 0.5 minute to 2 hours, preferably, from 20 minutes to 50 minutes.

In the present invention, the centrifugation temperature is not particularly limited. Preferably, the centrifugation is carried out at 1-10 °C, more preferably 2-6 °C.

In the present invention, the washing manner is not particularly limited. Preferably, the washing treatment is carried out in an environment of pH 7-8 (preferably, pH 7.4) by using a washing solution. The washing solution is not particularly limited, and a typical washing solution is selected from the group consisting of: potassium 4-hydroxyethylpiperazineethanesulfonate, potassium acetate, magnesium acetate, and combinations thereof.

In the present invention, the method for cell lysis treatment is not particularly limited. A preferred cell lysis treatment includes high-pressure lysis, freeze-thaw (e.g., treatment at liquid nitrogen low temperature) lysis, etc.

The mixture of nucleoside triphosphates in the *in vitro* protein synthesis system comprises adenosine triphosphate, guanosine triphosphate, cytidine triphosphate, and uridine triphosphate. **In** the present invention, there is no limitation to the concentration of each single nucleotide. Generally, the concentration of each single nucleotide is in a range of 0.5 mM to 5 mM, preferably in a range of 1.0 mM to 2.0 mM.

The amino acid mixture in the *in vitro* protein synthesis system may comprise natural or unnatural amino acids, and may include amino acids of D-type or amino acids of L-type. Representative amino acids include, but are not limited to, 20 types of natural amino acids: glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine. The concentration of each type of amino acid is usually in a range from 0.01 mM to 0.5 mM, preferably, from 0.02 mM to 0.2 mM, such as 0.05 mM, 0.06 mM, 0.07 mM, and 0.08 mM.

In a preferred embodiment, the *in vitro* protein synthesis system further comprises polyethylene glycol (PEG) or analogs thereof. The concentration of polyethylene glycol or analogs thereof is not particularly limited. Generally, the concentration (w/v) of polyethylene glycol or analogs thereof is in a range from 0.1% to 8%, preferably from 0.5% to 4%, more preferably from 1% to 2%, based on the total weight of the protein synthesis system. Representative examples of PEG include, but are not limited to, PEG3000, PEG8000, PEG6000 and PEG3350. It should be understood that the system according to the present invention may further comprise polyethylene glycol with other various molecular weights (such as PEG 200, 400, 1500, 2000, 4000, 6000, 8000, 10000 and so on).

In a preferred embodiment, the *in vitro* protein synthesis system further comprises sucrose. The concentration of sucrose is not particularly limited. Generally, the concentration of sucrose is in a range from 0.03 wt% to 40 wt%, preferably from 0.08 wt% to 10 wt%, more preferably from 0.1 wt% to 5 wt%, based on the total weight of the protein synthesis system.

In addition to the yeast extract, a particularly preferred *in vitro* protein synthesis system further comprises the following components: 22 mM 4-hydroxyethyl piperazineethanesulfonic acid (pH 7.4), 30-150 mM potassium acetate, 1.0-5.0 mM magnesium acetate, 1.5-4 mM nucleoside triphosphate mixture, 0.08-0.24 mM amino acid mixture, 25 mM phosphocreatine, 1.7 mM dithiothreitol, 0.27 mg/mL creatine phosphokinase, 1%-4% polyethylene glycol, 0.5%-2% sucrose, 8-20 ng/µL DNA of firefly luciferase and 0.027-0.054 mg/mL T7 RNA polymerase.

### CODING SEQUENCE OF EXOGENOUS PROTEINS (EXOGENOUS DNA)

As used herein, the terms "coding sequence of exogenous proteins" and "exogenous DNA" can be used interchangeably, and both refer to exogenous DNA molecules for guiding protein synthesis. The DNA molecules are generally linear or circular. The DNA molecules contain sequences encoding exogenous proteins. In the present invention, examples of sequences encoding exogenous proteins include, but are not limited to, genomic sequences and cDNA sequences. The sequences encoding exogenous proteins further comprise promoter sequence, 5' untranslated sequence, 3' untranslated sequence, or a combination thereof.

In the present invention, the selection of the exogenous DNA is not particularly limited. Generally, the exogenous DNA is selected from DNAs encoding luciferin, or luciferase (e.g., firefly luciferase), green fluorescent protein, yellow fluorescent protein, aminoacyl tRNA synthetase, glyceraldehyde-3-phosphate dehydrogenase, catalase, actin, variable regions of antibodies, DNA of a luciferase mutant, and any combination thereof.

The exogenous DNA may also be selected from exogenous DNAs encoding α-amylase, enterocin A, Hepatitis C virus E2 glycoprotein, insulin precursors, interferon αA, interleukin-1β, lysozyme, serum albumins, single-chain variable fragment (scFv) of antibodies, transthyretin, tyrosinase, xylanase, and any combination thereof.

In a preferred embodiment, the exogenous DNA encodes a protein which is selected from the group consisting of: green fluorescent protein (enhanced GFP, eGFP), yellow fluorescent protein (YFP), *E. coli* β-galactosidase (LacZ), human lysine-tRNA synthetase, human leucine-tRNA synthetase, Arabidopsis thaliana glyceraldehyde-3-phosphate dehydrogenase, murine catalase, and any combination thereof.

### HIGH-THROUGHPUT IN VITRO PROTEIN SYNTHESIS METHOD (METHOD FOR IMPROVING IN VITRO PROTEIN TRANSLATION EFFICIENCY)

The present invention provides a high-throughput method for *in vitro* protein synthesis, comprising steps of:
(i) providing the *in vitro* cell-free protein synthesis system according to the first aspect of the present invention, and adding exogenous DNA molecules for guiding protein synthesis, wherein the content of the EXN53 protein in the protein synthesis system is equal to or less than 10%, preferably equal to or less than 5%, and more preferably equal to or less than 2%; and
(ii) incubating the protein synthesis system provided in the step (i) for a period of time T1 under suitable conditions to synthesize proteins encoded by the exogenous DNA.

In a preferred embodiment, the present invention provides a design and a method for improving the *in vitro* protein translation efficiency by knocking-out the nuclease gene in the yeast genome, comprising the following steps:
1. design scheme and analysis method for enhancing *in vitro* biosynthesis activity is as follows:
   A. analysis of the impact of components in the *in vitro* biosynthesis system on the activity;
   B. analysis of the function and distribution of the nuclease gene in the *K. lactis* genome;
   C. modification design of a selected nuclease gene in the *K*. *lactis* genome;
2. construction of a cloning vector which has undergone treatment of targeted knock-out of gene, wherein the method comprises:
   A. designing a sgRNA sequence that guides endonuclease cleavage for specific gene sequence;
   B. recombining the sgRNA sequence into a vector containing an endonuclease to obtain a first vector in which the sgRNA and the endonuclease are co-expressed;
3. construction of donor DNA which is used for knock-out of specific gene, wherein the method comprises:
   A. downloading the nucleotide sequence of a specific gene from the gene database, and constructing a second vector by using sequences located at 1000 bp upstream and downstream of the gene, respectively;
   B. amplifying the second vector with primers M13F and M13R, and the obtained PCR product is concentrated by ethanol precipitation to obtain the donor DNA;
4. simultaneously transforming the first vector and the donor DNA into yeast competent cells;
   Screen out monoclonal cells for enlargement culture, extract the yeast genome, amplify the knock-out site with designed primers, and obtain strains with specific gene having been knocked out after verification by sequencing the amplified PCR product;
5. Cell-free *in vitro* protein synthesis system

Prepare a yeast cell solution using yeast strain with specific gene having been knocked out, add the yeast cell solution into an *in vitro* protein translation system and let the mixture stand for a reaction lasting for 2 to 6 hours in an environment of 20-30 °C; the absorbance was read to detect the activity of firefly luciferase by using a multifunctional microplate reader (Perkin Elmer).

The main advantages of the present invention include as follows.
1. The invention provides a technical route and method universally used for regulating the *in vitro* biosynthesis activity by reducing the content of nucleases in the system to improve the stability of nucleic acids and by performing systemic analysis on and making specific modification to the nuclease genes in the *K. lactis* genome.
2. In the present invention, a lot of effort have been made to screen out nucleases from a large quantities of nucleases and five nucleases were screened out from many nucleases for the first time. Surprisingly, it is found that the down-regulation or knock-out of EXN53, one of the five nucleases, can improve the stability of nucleic acids, and can greatly enhance the efficiency of protein production of the *in vitro* protein synthesis system. Specifically, after the down-regulation or knock-out of EXN53, the luciferase activity of the Δ*KLEXN53* yeast strain in the IVTT system was twice or more folds (e.g.,2.46-folds) of that of wild-type strain.
3. For the first time, a *K. lactis* strain which is capable of significantly increase the *in vitro* protein synthesis activity was provided in the present invention.

The present invention will be further illustrated below in combination with specific examples (or embodiments). It should be understood that these examples (or embodiments) are provided solely for the purpose of illustration and should not to be regarded as limitations to the scope of the present invention. With respect to the experimental methods without specifically described conditions in the following examples (or embodiments), one person may generally follow conventional conditions, such as conditions described in Sambrook *et.al,* Molecular Cloning: A Laboratory Manual (New York: Cold Spring Harbor Laboratory Press, 1989), or follow the conditions recommended by the manufacturer. Unless otherwise stated, percentage and portions refer to percentage by weight and portions by weight.

Unless otherwise stated, materials and reagents used in the examples of the present invention are all commercially available products.

### Example 1 Improving the Stability of Nucleic Acids by Reducing the content of the Nuclease in an in vitro Biosynthesis System, and Performing Analysis on and Making Specific Modification to the Nuclease Gene in the K. lactis Genome.

### 1. Design principle for regulating in vitro biosynthesis activity by modifying the K. lactis genome

### 1.1 Analysis of in vitro biosynthesis systems

### 1.1.1 Analysis of components in different in vitro biosynthesis systems

The *in vitro* biosynthesis system is used for translation of exogenous proteins through carrying out cell lysis on different types of cells (including microorganisms, animals and plants) to extract cell lysates. In order to achieve functions of *in vitro* biosynthesis systems such as transcription, translation, protein folding, energy metabolism and others, the cell lysate must contain elements for energy regeneration and protein synthesis, including ribosome, aminoacyl-tRNA synthetase, translation initiation factors, translation elongation factors, ribosome releasing factors, nucleotide recycling enzymes, metabolic enzymes, molecular chaperones, foldases and so on. Substances that need to be added exogenously include amino acids, nucleotides, DNA template, energy substrates, cofactors, salt molecules, etc. The stability of different components in the system has an important impact on the reaction duration time and final protein yield. The exhaustion of certain substrates (such as ATP, cysteine, etc.) is an important reason for the termination of the reaction.

### 1.1.2 Analysis model of the impact of components in the in vitro biosynthesis system on the synthesis product;

In the *in vitro* translation system, the concentration value of components including energy metabolism substrates (phosphocreatine, etc.), substrates involved in mRNA synthesis (e.g., NTPs, etc), substrates involved in protein synthesis (e.g., amino acids, etc), phosphate and other components as well as the pH value, changes along with the proceeding of the reaction, finally leading to the termination of the reaction. Both supplementing corresponding components (e.g., NTPs, amino acids, etc) and stabilizing corresponding conditions (e.g., pH value, phosphate concentration, etc) by using different techniques, can effectively extend the reaction time of the *in vitro* translation system, thereby increasing the protein yield.

### 1.1.3 Analysis of the impact of nucleic acids and nucleases in the in vitro protein synthesis system on protein synthesis;

As an important substrate for protein translation, the stability of DNA template and mRNA transcribed from the DNA template have an important impact on the reaction time and yield of the *in vitro* translation system. After cells are lysated, the cell lysate is collected and used to construct an *in vitro* translation system. In addition to various components necessary for protein synthesis, the *in vitro* translation system also contains components that are unfavorable against the reaction such as nucleases, proteases, etc. In a system composed of various purified components, the efficiency of protein translation can be significantly increased due to the absence of inhibitory factors.

Meanwhile, the stability of nucleic acids in the *in vitro* translation system can be effectively raised by using different technical means (including knock-out of nuclease genes and addition of stabilizing factors), so that protein translation efficiency can be improved. Figure 1 shows a design route for increasing the stability of the nucleic acid by reducing the nuclease level in an *in vitro* biosynthesis system, and performing analysis on and making specific modification to the nuclease gene in the *K. lactis* genome.

### 1.2 Analysis of nuclease genes in the K. lactis genome

### 1.2.1 Classification and distribution of nuclease genes in the K. lactis genome;

Nuclease (also referred to as nuclear polymerase or polynucleotidase) is an enzyme that can hydrolyze the phosphodiester bond between nucleotides in the first step of nucleic acid degradation. The nuclease can be divided into categories of exonuclease and endonuclease, according to the action position of the nuclease. The exonuclease which hydrolyzes nucleotides one by one starting from the 3'-terminal is referred to as 3' to 5' exonuclease. The exonuclease which hydrolyzes nucleotides one by one starting from the 5'-terminal is referred to as 5' to 3' exonuclease. The endonuclease catalyzes and hydrolyzes phosphodiester bond in the polynucleotide. The nuclease can also be divided into categories of deoxyribonuclease (DNase) and ribonuclease (RNase). The deoxyribonuclease (DNase) acts upon DNA, and the ribonuclease (RNase) acts upon RNA. The nuclease can also be classified into 5'to 3' exonuclease and 3'to 5' exonuclease according to the action direction of the nuclease.

Through alignment analysis of gene functions in the database, *K*. *lactis* contains a total of 61 nucleases, distributed on six chromosomes of *K. lactis* A-F (as shown in Figure 2), wherein nucleases located at the A chromosome are: *KLSEN54, KLDNA2, KLTRM2, KLFCF1, KLDOM34, KLRAD2, KLRNH70, KLDIS3* and *KLNPP1.* Code for *KLSEN54* in the KEGG database is: KlLA0A00803g, code for *KLDNA2* in the KEGG database is: KlLA0A05324g, code for *KLTRM2* in the KEGG database is: KlLA0A05665g, code for *KLFCF1* in the KEGG database is: KlLA0A07018g, code for *KLDOM34* in the KEGG database is: KlLA0A08646g, code for *KLRAD2* in the KEGG database is: KlLA0A09427g, code for *KLRNH70* in the KEGG database is: KlLA0A10065g, code for *KLDIS3* in the KEGG database is: KlLA0A10835g, and code for *KLNPP1* in the KEGG database is: KlLA0A11374g.

Nuclease located at the B chromosome is: *KLOGG1;* and code for *KLOGG1* in the KEGG database is: KlLA0B05159g.

Nucleases located at the C chromosome are: *KLPOL2, KLRAD50, KLYSH1, KLRCL1, KLNGL2, KLMRE11, KLPOP3, KLMKT1, KLAPN1, KLRPP1* and *KLPOP2.* Code for *KLPOL2* in the KEGG database is: KlLA0C02585g, code for *KLRAD50* in the KEGG database is: KlLA0C02915g, code for *KLYSH1* in the KEGG database is: KlLA0C04598g, code for *KLRCL1* in the KEGG database is: KlLA0C05984g, code for *KLRCL2* in the KEGG database is: KlLA0C06248g, code for *KLMRE11* in the KEGG database is: KlLA0C06930g, code for *KLPOP3* in the KEGG database is: KlLA0C12199g, code for *KLMKT1* in the KEGG database is: KlLA0C13926g, code for *KLAPN1* in the KEGG database is: KlLA0C14256g, code for *KLRPP1* in the KEGG database is: KlLA0C14718g, and code for *KLPOP2* in the KEGG database is: KlLA0C18821g.

Nucleases located at the D chromosome are: *KLNUC1, KLRRP6, KLDBR1, KLRPS3, KLRPM2, KLSUV3, KLRAD1, KLIRE1* and *KLPOP1;* Code for *KLNUC1* in the KEGG database is: KlLA0D00440g, code for *KLRRP6* in the KEGG database is: KlLA0D01309g, code for *KLDBR1* in the KEGG database is: KlLA0D04466g, code for *KLRPS3* in the KEGG database is: KlLA0D08305g, code for *KLRPM2* in the KEGG database is: KlLA0D10483g, code for *KLSUV3* in the KEGG database is: KlLA0D12034g, code for *KLRAD1* in the KEGG database is: KlLA0D12210g, code for *KLIRE1* in the KEGG database is: KLLA0D13266g, and code for *KLPOP1* in the KEGG database is: KlLA0D19448g.

Nucleases located at the E chromosome are: *KLPOL3, KLDXO1, KLMUS81, KLPAN3, KLAPN2, KLRAI1, KLEXO1, KLREX4, KLPAN2* and *KLLCL3.* Code for *KLPOL3* in the KEGG database is: KlLA0E01607g, code for *KLDXO1* in the KEGG database is: KlLA0E02245g, code for *KLMUS81* in the KEGG database is: KlLA0E03015g, code for *KLPAN3* in the KEGG database is: KlLA0E08097g, code for *KLAPN2* in the KEGG database is: KlLA0E18877g, code for *KLRAI1* in the KEGG database is: KlLA0E12893g, code for *KLEXO1* in the KEGG database is: KlLA0E16743g, code for *KLREX4* in the KEGG database is: KlLA0E17865g, code for *KLPAN2* in the KEGG database is: KlLA0E18877g, and code for *KLLCL3* in the KEGG database is: KlLA0E19163g.

Nucleases located at the F chromosome are: *KLRAD17, KLPOP4, KLPOP5, KLRAD27, KLRNH201, KLVMA1, KLRAT1, KLNGL1, KLREX2, KLTRL1, KLPOL31, KLCCR4, KLTRZ1, KLSEN2, KLREX3, KLSWT1, KLEXN53, KLRNT1, KLSEN15, KLNTG1, KLNOB1* and *KLDDP1.* Code for *KLRAD17* in the KEGG database is: KlLA0F00330g, code for *KLPOP4* in the KEGG database is: KlLA0F02211g, code for *KLPOP5* in the KEGG database is: KlLA0F02453g, code for *KLRAD27* in the KEGG database is: KlLA0F02992g, code for *KLRNH201* in the KEGG database is: KlLA0F04774g, code for *KLVMA1* in the KEGG database is: KlLA0F05401g, code for *KLRAT1* in the KEGG database is: KlLA0F07469g, code for *KLNGL1* in the KEGG database is: KlLA0F07733g, code for *KLREX2* in the KEGG database is: KlLA0F08998g, code for *KLPOL31* in the KEGG database is: KlLA0F14949g, code for *KLCCR4* in the KEGG database is: KlLA0F15884g, code for *KLTRZ1* in the KEGG database is: KlLA0F16665g, code for *KLSEN2* in the KEGG database is: KlLA0F19866g, code for *KLREX3* in the KEGG database is: KlLA0F19910g, code for *KLSWT1* in the KEGG database is: KlLA0F20361g, code for *KLEXN53* in the KEGG database is: KlLA0F22385g, code for *KLRNT1* in the KEGG database is: KlLA0F24816g, code for *KLSEN15* in the KEGG database is: KlLA0F26334g, code for *KLNTG1* in the KEGG database is: KlLA0F07711g, code for *KLNOB1* in the KEGG database is: KlLA0F16280g, and code for *KLDDP1* in the KEGG database is: KlLA0F20273g.

The *K. lactis* nucleases can be classified into two categories according to the function. Wherein, there are 18 DNases and 41 RNases, and there are also two unclassified nucleases (as shown in Table 1). DNases having 3' to 5' function include KLAPN1, and DNases having 5' to 3' function include KLRAD27 and KLEX01; RNases having 3' to 5' function include KLRNH70, KLDIS3, KLNGL2 and KLRRP6, and RNases having 5' to 3' function include KLDXO1, KLRAT1 and KLEXN53.

**Table 1**

| Nuclease | Distribution of nuclease gene | Total Number |
|---|---|---|
| DNases | Chromosome A: *KLDNA2, KLNPP1, KLRAD2;* | 18 |
| | Chromosome B: *KLOGG1;* | |
| | Chromosome C: *KLPOL2, KLRAD50, KLMRE11, KLAPN1;* | |
| | Chromosome D: *KLRPS3, KLRAD1;* | |
| | Chromosome E: *KLPOL3, KLMUS81, KLAPN2, KLEXO1*; | |
| | Chromosome F: *KLRAD27, KLRAD17, KLPOL31, KLNTG1;* | |
| RNases | Chromosome A: *KLSEN54, KLTRM2, KLFCF1, KLRNH70, KLDIS3;* | 41 |
| | Chromosome *C:KLYSH1, KLRCL1, KLNGL2, KLPOP3, KLMKT1, KLRPP1, KLPOP2;* | |
| | Chromosome D: *KLNUC1, KLRRP6, KLDBR1, KLRPM2, KLSUV3, KLIRE1, KLPOP1*: | |
| | Chromosome E: *KLDXO1, KLPAN3, KLRAI1, KLREX4, KLPAN2;* | |
| | Chromosome *F:KLPOP4, KLPOP5, KLRNH201, KLVMA1, KLRAT1, KLNGL1, KLREX2, KLCCR4, KLTRZ1, KLSEN2, KLREX3, KLSWT1, KLEXN53, KLRNT1, KLSEN15, KLNOB1, KLDDP1;* | |
| No category | *KLDOM34, KLLCL3;* | 2 |

### 1.2.2 Analysis for selection of nuclease genes in the K. lactis genome involved in the in vitro protein synthesis;

Nucleic acids of RNA and DNA are coding substrates of the *in vitro* protein synthesis system, and their stability affects protein yield. During the process of eukaryotic translation, the 5'-terminal of RNA can instantly complete the processing of a cap structure, and the cap structure at the 5'-terminal of RNA plays an important role in RNA stability and translation efficiency.

In eukaryotes, there exist deadenylation-dependent RNA degradation mechanism and adenylation-independent RNA degradation mechanism. In the mechanism of deadenylation-dependent RNA degradation, the mature cap structure or immature cap structure at the 5'-terminal of RNA can be removed by recruiting a cap-removing complex, and meanwhile the RNA undergoes degradation in a direction from 5' to 3' under the action of nuclease. In the mechanism of adenylation-independent mRNA degradation, the nucleic acid molecule is degraded into broken strands from the middle position under the action of endonuclease, and the exonuclease degrades RNA in a direction from 3' to 5'.

In the *K. lactis* based *in vitro* protein synthesis system, exogenous linear or circular DNA used as template can be transcribed into mRNA with a 3' polyA tag; depending on the difference of promoters and RNA transcriptases, the transcribed mRNA may have or do not have a 5' mature cap structure. Therefore, DNases, RNases, exonucleases and endonucleases will all affect the stability of the template in the *in vitro* protein synthesis system, and the modification of those enzymes may generate an enhancing effect on the *in vitro* biosynthesis activity.

Wherein, due to the special instability of mRNA and the high activity of *in vivo* RNase, the lifespan of mRNA is a main bottleneck restricting protein synthesis *in vitro.* Reducing *in vivo* RNases via modification is the most preferred modification scheme. Of course, it should be noted that the present invention is still applicable to designs for other nucleases, so as to improve the *in vitro* protein biosynthesis activity.

### 1.2.3 Specific modification design of nuclease genes (EXN53, Rat 1, Rrp6, Dis3 and NGL2) in the K. lactis genome;

In *K. lactis,* 5' to 3' exonucleases include EXN53 and Rat 1, wherein EXN53 is located in the cytoplasm and Rat 1 is located in the nucleus. In the mechanism of deadenylation-dependent RNA degradation, after a process of deadenylation, RNA can be degraded by protein subunit complex (exosome) which is located in both cytoplasm and nucleus, and has both 3' to 5' exonuclease activity and endonuclease activity. In *K. lactis,* 3' to 5' exonucleases include Rrp6, Dis3 (also referred to as Rrp44), NGL2, and so on. Wherein Rrp6 and Dis3 are both part of the protein subunit complex (exosome) which has both exonuclease activity and endonuclease activity. Rrp6 is a kind of 3' to 5' exonuclease and a member of the RNase D family; Rrp6 is located in the nucleus, and is a key catalytic subunit of the exosome in the nucleus. In addition, Rrp6 is responsible for the processing, degradation and quality control of various RNAs in the cell. Dis3 (also referred to as Rrp44) is a key catalytic subunit of exosome, and a member of the highly conserved RNaseII/RNB family; Dis3 is located in both nucleus and cytoplasm. Furthermore, Dis3 has both 3' to 5' exonuclease activity and endonuclease activity, and is responsible for the metabolism of RNA and the processing of all types of RNAs in both cytoplasm and nucleus. NGL2 is an enzyme that specifically recognizes Poly A, which hydrolyzes RNA in a direction from 3' to 5'.

Based on the above analysis, five genes of *EXN53, Rat1, Rrp6, Dis3* and *NGL2* were selected to be modified in the present invention by using relevant methods including, but not limited to, gene knock-out and gene mutation. Improve the stability of nucleic acids via downregulating the nuclease in an *in vitro* biosynthesis system and by virtue of a design for performing analysis on and making specific modification to the nuclease gene in the *K. lactis* genome; improve the *in vitro* protein translation efficiency by virtue of the design and technical details for regulating the *in vitro* protein synthesis activity.

### 1.3 Analysis of method for targeted gene alteration in the K. lactis genome;

### 1.3.1 Method for modification of the K. lactis genome and selection analysis

In the present invention, the *K. lactis* genome was modified via CRISPR-Cas9 gene editing technology. CRISPR is a widely used gene editing technology, and has advantages of structural simplicity, convenient operation, high editing efficiency, achievement of knocking-out multiple sites of the target gene in the meantime without introduction of antibiotics, and so on. Relatively mature transformation systems have already been built in the yeast.

### 1.3.2 Technical design of CRISPR-Cas9 for K. lactis genome

The CRISPR system used in the present invention is an optimized system for the *K. lactis* strain, in which parameters and conditions of coding sequences of Cas9 proteins, gRNA promoters, length of homologous arms of donor DNA, preparation and transformation of yeast competents, and so on were optimized, and efficient editing in the *K. lactis* strain was achieved.

### 1.3.3 Design of CRISPR/Cas9 for knock-out of K. lactis nuclease genes (EXN53, Rat1, Rrp6, Dis3 and NGL3) in the K. lactis genome

In the present invention, two homologous arms of the donor DNA are gene sequences located at about 1,000 bp upstream and downstream of the ORF of the nuclease gene to be knocked out, respectively; constructing two gRNAs which respectively recognize the 5'- and 3' PAM sequences of the ORF of the nuclease gene to be knocked out at the same time and guide Cas9 nuclease cleave DNA in a site-specific manner, then homologous recombination happens after DSB (double-strand break) occurs, thereby achieving knock-out of nuclease gene. For example, the knock-out of the 5 genes of EXN53, Rat1, Rrp6, Dis3 and NGL3 were carried out by placing one gRNA near the initiation codon and one gRNA near the termination codon respectively. The CRISPR system designed for the modification of the five nuclease genes in the *K. lactis* genome is shown in Figure 3.

### Example 2 Targeted Knock-out of EXN53 Gene via CRISPR-Cas9

### 2.1 Search for KLEXN53 sequence and determination of CRISPR gRNA sequence

### 2.1.1 Construction of cloning vector plasmid for targeted knock-out of gene:

In KEGG database, BLAST alignment analysis was carried out based on *EXN53* gene. As a result, the gene sequence (SEQ ID NO.1) of *EXN53* in *Kluyveromyces lactis* which has a code of K1LA0F22385g in the KEGG database was determined. The determined gene sequence has a 60.93% gene sequence homology and a 57.65% protein sequence homology to *EXN53* in *S. cerevisiae,* has a 47.38% gene sequence homology and a 38.29% protein sequence homology to *EXN53* in *S. pombe,* and has a 43.81% gene sequence homology and a 33.48% protein sequence homology to *EXN53* in *H. sapiens* (as shown in Figure 5). The determined gene sequence has characteristic EXN53 5' to 3' exonuclease domain and Amelogenin domain (as shown in Figure 4). The gene is named *KLEXN53* (located at 2091235 ... 2095596 of chromosome F).

The PAM sequence (NGG) was searched for at the initiation codon and the termination codon of the *KLEXN53* gene, and gRNA sequences were identified. The principle for selecting the gRNA is as follows: the GC content should be moderate (wherein, the standard in the present invention is that the GC content is in a range of 40%-60%), and the existence of a poly T structure should be avoided. Finally, the *KLEXN53* gRNA-1 sequence determined by the present invention was AGAGTTCGACAATTTGTACT (SEQ ID No.:95), and the *KLEXN53* gRNA-2 sequence determined by the present invention was CGTCGTGGCCGTAGTAATCG (SEQ ID No.:96).

Method for construction and transformation of plasmids is as follows: two PCR amplification processes were carried out by using a pair of primers pCas9-KLEXN53-F1: AGAGTTCGACAATTTGTACTGTTTTAGAGCTAGAAATAGCAAGT TAAAATAAGGCTAGTC (SEQ ID NO.:7) and pCas9-KLEXN53-R1: GCTCTAAAACAGTACAAATTGTCGAACTCTAAAGTCCCATTCGC CACCCG (SEQ ID NO.:8) with a pCAS plasmid as template, and by using a pair of primers pCas9-KLEXN53-F2: CGTCGTGGCCGTAGTAATCGGTTTTAGAGCTAGAAATAGCAAGT TAAAATAAGGCTAGTC(SEQ ID NO.:9) and pCas9- KLEXN53-R2: GCTCTAAAACCGATTACTACGGCCACGACGAAAGTCCCATTCGC CACCCG(SEQ ID NO.:10) with a pCAS plasmid as template, respectively. 17 µL product of each PCR amplification process were mixed followed by the addition of 1 µL of Dpn I (DpnI) and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored, and named pHoCas9_SE_Kana_tRNA_ScRNR2_KLEXN53-1 and pHoCas9_SE_Kana_tRNA_ScRNR2_KLEXN53-2, respectively. One PCR amplification process was carried out by using primers of pCas9-F1: TAGGTCTAGAGATCTGTTTAGCTTGCCTCG(SEQ ID NO.:11) and pCas9-R: TATCCACTAGACAGAAGTTTGCGTTCC (SEQ ID NO.:12) with pHoCas9_SE_Kana_tRNA_ScRNR2_KLEXN53-1 as template, and another PCR amplification process was carried out by using primers of pCas9-F2:TATGGAACGCAAACTTCTGTCTAGTGGATAGTATATGT GTTATGTAGTATACTCTTTCTTCAACAATTAAATACTCTCGG (SEQ ID NO.:13) and pCas9-F2: CGAGGCAAGCTAAACAGATCTCTAGACCTATATCCACTAGACAG AAGTTTGCGTTCC(SEQ ID NO.:14) with pHoCas9_SE_Kana_tRNA_ScRNR2_KLEXN533-2 as template. PCR amplification products of pCas9-F1/pCas9-R1 and pCas9-F2/pCas9-R2 were mixed at a ratio of 1:5, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored, and named pHoCas9_SE_Kana_tRNA_ScRNR2_KLEXNS53-1&2, respectively (as shown in Figure 10).

### 2.2 Construction and amplification of donor DNA plasmid

To facilitate the storage and amplification of linear donor DNA, the donor DNA was firstly inserted into a pMD18 plasmid, and then amplified by PCR to obtain a linear donor DNA sequence in the present invention.

One PCR amplification process was carried out by using primers of KLEXN53-F1: GTACCCGGGGATCCTCTAGAGATCCAGTTGCAGAGCCTCCGAA (SEQ ID NO.:15) and KLEXN53-R1: CATGCCTGCAGGTCGACGATGCGAAACCTTAGCTCTTTATCGAA C (SEQ ID NO.:16) with a *Kluyveromyces lactis* genomic DNA as template; another PCR amplification was carried out by using primers of pMD18-F: ATCGTCGACCTGCAGGCATG (SEQ ID NO.:17) and pMD18-R: ATCTCTAGAGGATCCCCGGG (SEQ ID NO.:18) with a pMD18 plasmid as template. Products of the two PCR amplification processes (8.5 µL for each PCR product) were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmid was extracted and stored, named KLEXN53-pMD18.

PCR amplification was carried out by using primers of KLEXN53-F2:
ATGTTGGTTTGAATGGACTATTAACAGTAAATATTATATCACTTC (SEQ ID NO.:19) and KLEXN53-R2: GAAGTGATATAATATTTACTGTTAATAGTCCATTCAAACCAACAT TTATTTTAGTTAAGCCACAAACCGTAATTAATTGAACAC (SEQ ID NO.:20) with a KLEXN53-pMD18 plasmid as template; a plasmid of KLEXN53-DD-pMD18 was constructed (as shown in Figure 11). Specific steps include as follows: the two PCR products (8.5 µL for each PCR product) were mixed, followed by the addition of 1 µL of Dpn I and 2 µL 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Five monoclonal colonies were picked out and then cultured with shaking in an LB liquid medium. After they were tested positive by PCR and confirmed by sequencing, the plasmid was extracted and stored.

### 2.3 Transformation and positive identification of Kluyveromyces lactis

2.3.1 A *Kluyveromyces lactis* solution was streaked and cultured on YPD solid medium. Monoclonal colonies were picked out and then cultured with shaking overnight in 25 mL 2×YPD liquid medium. 2 mL of the yeast solution was taken and further cultured with shaking in 50 mL 2×YPD liquid medium for 2 to 8 hours. Subsequently, yeast cells were collected by centrifugation at 3,000 g for 5 minutes under the condition of 20°C, and resuspended with the addition of 500 µL of sterile water. Similarly, cells were collected by centrifugation under the same condition. A competent cell solution (5% v/v glycerol and 10% v/v DMSO) was prepared and the yeast cells were dissolved in 500 µL of this solution. 50 µL of aliquots were aliquoted into 1.5 mL centrifuge tubes and stored at -80 °C.

2.3.2 Competent cells were placed at 37 °C for thawing for 15 to 30 seconds, centrifuged at 13,000 g for 2 minutes, and the supernatant was removed. Transformation buffer solution was prepared as follows: 260 µL of PEG 3350 (50% (w/v)), 36 µL of LiAc (1.0 M), 20 µL of carrier DNA (5.0 mg/mL), 5 µL of Cas9/gRNA plasmid and 10 µL of donor DNA were added; then sterile water was added to form a mixture of a final volume of 360 µL. After heatshock, centrifugation was carried out at 13,000 g for 30 seconds to remove the supernatant. 1 mL of YPD liquid medium was added, and the mixture was incubated for 2 to 3 hours. 200 µL of the mixture was pipetted and coated onto an YPD (200 µg/mL G418) solid medium and then incubated for 2 to 3 days until monoclonal colonies grew out.

2.3.3 10 to 20 monoclonal colonies were picked out from a plate with transformed *Kluyveromyces lactis* cells. The monoclonal colonies were placed in 1 mL YPD (200 µg/mL G418) and then cultured with shaking overnight. Samples were detected by PCR using CRISPR Insertion Check primers of KLEXN53-CICF1: TTTGCTGGTTGCCCGTATTCCC (SEQ ID NO.:21) and KLEXN53-CICR1: TAATAGCACAGGGAATGCACCTT (SEQ ID NO.:22) with the yeast solution as template. Strains that were tested positive by PCR and identified by sequencing, were determined as positive strains.

### Comparative Example 3 Targeted Knock-out of DIS3 Gene via CRISPR-Cas9

### 3.1 Search for KLDIS3 sequence and determination of CRISPR gRNA sequence

### 3.1.1 Construction of cloning vector plasmid for targeted knock-out of gene:

In KEGG database, BLAST alignment analysis was carried out based on *DIS3* gene. As a result, the gene sequence (SEQ ID NO.2) of *DIS3* in *Kluyveromyces lactis* which has a code of K1LA0A10835g in the KEGG database was determined. The determined gene sequence has a 70.54% gene sequence homology and a 77.59% protein sequence homology to *DIS3* in *S. cerevisiae,* has a 56.86% gene sequence homology and a 51.82% protein sequence homology to *DIS3* in *S. pombe,* and has a 48.83% gene sequence homology and a 40.19% protein sequence homology to *DIS3* in *H. sapiens* (as shown in Figure 6). The determined gene sequence has characteristic VacB domain and PIN_Rrp44 domain (as shown in Figure 4). The gene is named *KLDIS3* (located at 938712...941738 of chromosome A).

The PAM sequence (NGG) was searched for nearby the initiation codon and the termination codon of the *KLDIS3* gene, and gRNA sequences were identified. The principle for selecting the gRNA is as follows: the GC content should be moderate (wherein, the standard in the present invention is that the GC content herein is in a range of 40%-60%), and the existence of a poly T structure should be avoided. Finally, the *KLDIS3* gRNA-1 sequence determined by the present invention was TTCAGCAGCTAAGAAGGAAG (SEQ ID NO.:23), and the *KLDIS3* gRNA-2 sequence determined by the present invention was AGAGGTCAGTGTCTTTGATA (SEQ ID NO.:24).

Method for construction and transformation of plasmids is as follows: two PCR amplification processes were carried out by using a pair of primers pCas9-KLDIS3-F1:
ATGGGACTTTTTCAGCAGCTAAGAAGGAAGGTTTTAGAGCTAG AAATAGCAAGTTAAAATAAGGCTAGTCC (SEQ ID NO.:25) and pCas9-KLDIS3-R1:
AGCTCTAAAACCTTCCTTCTTAGCTGCTGAAAAAGTCCCATTCG CCACCCG (SEQ ID NO.:26) with a pCAS plasmid as template, and by using a pair of primers pCas9-KLDIS3-F2: ATGGGACTTTAGAGGTCAGTGTCTTTGATAGTTTTAGAGCTAGA AATAGCAAGTTAAAATAAGGCTAGTCC (SEQ ID NO.:27) and pCas9-KLDIS3-R2:
AGCTCTAAAACTATCAAAGACACTGACCTCTAAAGTCCCATTCG CCACCCG (SEQ ID NO.:28) with a pCAS plasmid as template, respectively. 17 µL product of each PCR amplification process were mixed, followed by the addition of 1 µL of Dpn I and 2 µL 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored, and named pHoCas9_SE_Kana_tRNA_ScRNR2_KLDIS3-1 and pHoCas9_SE_Kana_tRNA_ScRNR2_ KLDIS3-2, respectively. One PCR amplification process was carried out using primers pCas9-F1: TAGGTCTAGAGATCTGTTTAGCTTGCCTCG (SEQ ID NO.:29) and pCas9-R1: TATCCACTAGACAGAAGTTTGCGTTCC (SEQ ID NO.:30) with pHoCas9_SE_Kana_tRNA_ScRNR2_KLDIS3-1 as template, and another PCR amplification process was carried out using primers pCas9-F2:
TATGGAACGCAAACTTCTGTCTAGTGGATAGTATATGTGTTATGT AGTATACTCTTTCTTCAACAATTAAATACTCTCGG (SEQ ID NO.:31) and pCas9-R2: CGAGGCAAGCTAAACAGATCTCTAGACCTATATCCACTAGACAG AAGTTTGCGTTCC (SEQ ID NO.:32) with pHoCas9_SE_Kana_tRNA_ScRNR2_KLDIS3-2 as template. PCR products of pCas9-F1/pCas9-R1 and pCas9-F2/pCas9-R2 were mixed at a ratio of 1:5, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored, and named pHoCas9_SE_Kana_tRNA_ScRNR2_KLDIS-1&2, respectively (as shown in Figure 12).

### 3.2 Construction and amplification of donor DNA plasmid

To facilitate the storage and amplification of linear donor DNA, the donor DNA was firstly inserted into a pMD18 plasmid, and then amplified by PCR to obtain a linear donor DNA in the present invention.

One PCR amplification process was carried out using primers KLDIS3-F1:
CCCGGGGATCCTCTAGAGATGCTGCTAGGTGACAGAAGGTTGT CC (SEQ ID NO.:33) and KLDIS3-R1: CATGCCTGCAGGTCGACGATCCAAAGAAGAACGTCGTAAGACC GC (SEQ ID NO.:34) with a *Kluyveromyces lactis* genomic DNA as template; another PCR amplification process was carried out using primers of pMD18-F: ATCGTCGACCTGCAGGCATG (SEQ ID NO.:35) and pMD18-R: ATCTCTAGAGGATCCCCGGG (SEQ ID NO.:36) with a pMD18 plasmid as template. Products of the two PCR amplification processes (8.5 µL for each PCR product) were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored, and named KLDIS3-pMD18.

PCR amplification was carried out using primers of KLDIS3-F2: CTCTTCTGTTTAGCACCCGGTTATAGCTTAATTTATTAATTATGTA CATTATATAAAAACTATTGTC (SEQ ID NO.:37) and KLDIS3-R2: AAGCTATAACCGGGTGCTAAACAGAAGAGTATGACGTTTTATAC TTCTCCAG (SEQ ID NO.:38) with a KLDIS3- pMD18 plasmid as template; a KLDIS3-DD-pMD18 plasmid was constructed (as shown in Figure 13). Specific steps include as follows: the two PCR products (8.5 µL for each PCR product) were mixed, followed by the addition of 1 µL of Dpn I and 2 µL 10 × digestion buffer, and the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored.

### 3.3 Transformation and positive identification of Kluyveromyces lactis

3.3.1 A *Kluyveromyces lactis* solution was streaked and cultured on YPD solid medium. Monoclonal colonies were picked out, and then the cultured with shaking overnight in 25 mL 2×YPD liquid medium. 2 mL of the yeast solution was taken and further cultured with shaking in 50 mL 2×YPD liquid medium for 2 to 8 hours. Subsequently, yeast cells were collected by centrifugation at 3,000 g for 5 minutes under the condition of 20 °C, and resuspended with the addition of 500 µL of sterile water. Similarly, cells were collected by centrifugation under the same condition. A competent cell solution (5% v/v glycerol and10% v/v DMSO) was prepared and the yeast cells were dissolved in 500 µL of this solution. 50 µL of liquots were aliquoted into 1.5 mL centrifuge tubes and stored at -80 °C.

3.3.2 Competent cells were placed at 37 °C for thawing for 15 to 30 seconds, centrifuged at 13,000 g for 2 minutes, and the supernatant was removed. Transformation buffer solution was prepared as follows: 260 µL of PEG 3350 (50% (w/v)), 36 µL of LiAc (1.0 M), 20 µL of carrier DNA (5.0 mg/mL), 15 µL of Cas9/gRNA plasmid, 10 µL of donor DNA were added; then sterile water was added to form a mixture of a final volume of 360 µL. After heatshock, centrifugation was carried out at 13,000 g for 30 seconds to remove the supernatant. 1 mL of YPD liquid medium was added to the solution, and the mixture was incubated for 2 to 3 hours. 200 µL of the mixture was pipetted and coated onto an YPD (200 µg/mL G418) solid medium and then incubated for 2 to 3 days until monoclonal colonies grew out.

3.3.3 10 to 20 monoclonal colonies were picked out from a plate with transformed *Kluyveromyces lactis* cells. The monoclonal colonies were placed in 1 ml YPD (200 µg/mL G418) and cultured with shaking overnight. Samples were detected by PCR using CRISPR Insertion Check primers of KLDIS3-CICF1: CACCAACAACAGGAAATCTCATG (SEQ ID NO.:39) and KLDIS3-CICR1: CAGTACAGAA GCTCAGCAAC AACC (SEQ ID NO.:40) with the yeast solution as template. Strains that were tested positive by PCR and identified by sequencing, were determined as positive strains.

### Comparative Example 4 Targeted Knock-out of Rat1 Gene via CRISPR-Cas9

### 4.1 Search for KLRat1 sequence and determination of CRISPR gRNA sequence

### 4.1.1 Construction of cloning vector plasmid for targeted knock-out of gene:

In KEGG database, BLAST alignment analysis was carried out based on the *Rat1* gene. As a result, the gene sequence (SEQ ID NO.3) of *Rat1* in *Kluyveromyces lactis* which has a code of K1LA0F07469g in the KEGG database was determined. The determined gene sequence has a 60.38% gene sequence homology and a 62.30% protein sequence homology to *Rat1* in *S. cerevisiae,* has a 50.53% gene sequence homology and a 39.73% protein sequence homology to *Rat1* in *S. pombe,* and has a 50.15% gene sequence homology and a 41.41% protein sequence homology to *Rat1* in *H .sapiens* (as shown in Figure 7). The determined gene sequence has a characteristic *EXN535 5'* to 3' exonuclease domain (as shown in Figure 4). The gene is named *KLRat1* (located at 703955...706933 of chromosome F).

The PAM sequence (NGG) was searched for nearby the initiation codon and the termination codon of the Rat1 gene, and gRNA sequences were identified. The principle for searching the gRNA is as follows: the GC content should be moderate (wherein, the standard in the present invention is that the GC content is in a range of 40%-60%) and the existence of a poly T structure should be avoided. Finally, the *KLRat1* gRNA-1 sequence determined by the present invention was GTAAGGCCAGGTACTCACAA (SEQ ID NO.:41), and *KLRat1* gRNA-2 sequence determined by the present invention was CTCGCAACAGAGACAGCCAC (SEQ ID NO.:42).

Method for construction and transformation of plasmids is as follows: two PCR amplification processes were carried out by using a pair of primers pCas9-KLRat1-F1:ATGGGACTTTGTAAGGCCAGGTACTCACAAGT TTTAGAGCTAGAAATAGCAAGTTAAAATAAGGCTAGTCC (SEQ ID NO.:43) and pCas9-KLRat1-R1: AGCTCTAAAACTTGTGAGTACCTGGCCTTACAAAGTCCCATTCG CCACCCG (SEQ ID NO.:44) with a pCAS plasmid as template, and by using a pair of primers pCas9-KLRat1-F2: ATGGGACTTTCTCGCAACAGAGACAGCCACGTTTTAGAGCTAG AAATAGCAAGTTAAAATAAGGCTAGTCC (SEQ ID NO.:45) and pCas9-KLRat1-R2:
AGCTCTAAAACGTGGCTGTCTCTGTTGCGAGAAAGTCCCATTCG CCACCCG (SEQ ID NO.:46) with a pCAS plasmid as template, respectively. 17 µL product of each PCR amplification process were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named pHoCas9_SE_Kana_tRNA_ScRNR2_KLRat1-1 and pHoCas9_SE_Kana_tRNA_ScRNR2_KLRat1-2, respectively.

One PCR amplification process was carried out by using primers of pCas9-F1: TAGGTCTAGAGATCTGTTTAGCTTGCCTCG (SEQ ID NO.:47) and pCas9-R1: TATCCACTAGACAGAAGTTTGCGTTCC (SEQ ID NO.:48) with pHoCas9_SE_Kana_tRNA_ScRNR2_KLRat1-1 as template. Another PCR amplification process was carried out by using primers of pCas9-F2: TATGGAACGCAAACTTCTGTCTAGTGGATAGTATATGTGTTATGT AGTATACTCTTTCTTCAACAATTAAATACTCTCGG (SEQ ID NO.:49) and pCas9-R2: CGAGGCAAGCTAAACAGATCTCTAGACCTATATCCACTAGACAG AAGTTTGCGTTCC (SEQ ID NO.:50) with pHoCas9_SE_Kana_tRNA_ScRNR2_KLDIS3-2 as template. PCR amplification products of pCas9-F1/pCas9-R1 and pCas9-F2/ pCas9-R2 were mixed at a ratio of 1:5, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named pHoCas9_SE_Kana_tRNA_ScRNR2_KLRat1-1&2, respectively (as shown in Figure 14).

### 4.2 Construction and amplification of donor DNA plasmid

To facilitate the storage and amplification of linear donor DNA, the donor DNA was firstly inserted into a pMD18 plasmid in the present invention, and then amplified by PCR to obtain a linear donor DNA sequence.

One PCR amplification process was carried out by using primers of KLRat1-F1: CCCGGGGATCCTCTAGAGATGCTGCATGGTCACAGGAGATGC (SEQ ID NO.:51) and KLRat1-R1: CATGCCTGCAGGTCGACGATGGTACGTGAGGCGACAATATGGTC C (SEQ ID NO.:52) with a *Kluyveromyces lactis* genomic DNA as template; another PCR amplification process was carried out by using primers of pMD18-F: ATCGTCGACCTGCAGGCATG (SEQ ID NO.:53) and pMD18-R: ATCTCTAGAGGATCCCCGGG (SEQ ID NO.:54) with a pMD18 plasmid as template. Products of the two PCR amplification processes (8.5 µL for each PCR product) were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated on an Amp-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named KLRat1-pMD18.

PCR amplification was carried out by using primers of KLRat1-F2:
CCAGGTACTCACATGAACTGTGGACAATTTTATACCCGTTTATAT CAGCAC (SEQ ID NO.:55) and KLRat1-R2: TGTCCACAGTTCATGTGAGTACCTGGCCTTACTTCTCGC (SEQ ID NO.:56) with a KLRat1-pMD18 plasmid as template; KLRat1-DD-pMD18 was constructed (as shown in Figure 15). Specific steps include as follows: the two PCR products (8.5 µL for each PCR product) were mixed, followed by the addition of 1 µL of Dpn I and 2 µL 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored.

### 4.3 Transformation and positive identification of Kluyveromyces lactis

4.3.1 A *Kluyveromyces lactis* solution was streaked and cultured on YPD solid medium. Monoclonal colonies were picked out, and then cultured with shaking overnight in 25 mL 2×YPD liquid medium. 2 mL of the yeast solution was taken and further cultured with shaking in the 2×YPD liquid medium for 2 to 8 hours. Subsequently, yeast cells were collected by centrifugation 3,000 g for 5 minutes under the condition of 20 °C, and resuspended with the addition of 500 µL of sterile water. Similarly, cells were collected by centrifugation under the same condition. A competent cell solution (5% v/v glycerol and10% v/v DMSO) was prepared and the yeast cells were dissolved in 500 µL of this solution. 50 µL of aliquots were aliquoted into 1.5 mL centrifuge tubes and stored at -80°C.

4.3.2 Competent cells were placed at 37 °C for thawing for 15 to 30 seconds, centrifuged at 13,000 g for 2 minutes, and the supernatant was removed. Transformation buffer solution was prepared as follows: 260 µL of PEG 3350 (50% (w/v)), 36 µL of LiAc (1.0 M), 20 µL of carrier DNA (5.0 mg/mL), 15 µL of Cas9/gRNA plasmid, 10 µL of donor DNA were added; then sterile water was added to form a mixture of a final volume of 360 µL. After heatshock, centrifugation was carried out at 13,000 g for 30 seconds to remove the supernatant. 1 ml of YPD liquid medium was added, and the mixture was incubated for 2 to 3 hours. 200 µL of the mixture was pipetted and coated onto an YPD (200 µg/mL G418) solid medium and then incubated for 2 to 3 days until monoclonal colonies grew out.

4.3.3 10 to 20 monoclonal colonies were picked out from a plate with transformed *Kluyveromyces lactis* cells. The monoclonal colonies were placed in 1 mL YPD (200 µg/mL G418) and cultured with shaking overnight. Samples were detected by PCR using CRISPR Insertion Check primers of KLRat1-CICF1: TAAGACAGGTACCCTCACGACG (SEQ ID NO.:57) and KLRat1-CICR1: CTTGGAAGTGGATACATTTCTAGAGG (SEQ ID NO.:58) with the yeast solution as template. Strains that were tested positive by PCR and identified by sequencing, were determined as positive strains.

### Comparative Example 5 Targeted Knock-out of Rrp6 Gene by CRISPR-Cas9

### 5.1 Search for KLRrp6 sequence and determination of CRISPR gRNA sequence

### 5.1.1 Construction of cloning vector plasmid for targeted knock-out of gene:

In KEGG database, BLAST alignment analysis was carried out based on *Rrp6* gene. As a result, the gene sequence (SEQ ID NO.4) of *Rrp6* in *Kluyveromyces lactis* which has a code of K1LA0D01309g in the KEGG database was determined and was referred to as a hypothetical protein. The determined gene sequence has a 55.04% gene sequence homology and a 46.93% protein sequence homology to *Rrp6* in *S. cerevisiae,* has a 44.04% gene sequence homology and a 29.85% protein sequence homology to *Rrp6* in *S. pombe,* and has a 40.44% gene sequence homology and a 23.53% protein sequence homology to *Rrp6* in *H. sapiens* (as shown in Figure 8). The determined gene sequence has characteristic Rrp6p-like exonuclease domain, PMC2NT domain as well as Helicase and RNase D C-terminal domain (as shown in Figure 4). The gene is named *KLRrp6* (located at 114713...116947 of chromosome D).

The PAM sequence (NGG) was searched for nearby the initiation codon and the termination codon of the *Rrp6* gene, and gRNA sequences were determined. The principle for selecting the gRNA is as follows: the ratio of GC content should be moderate (wherein, the standard in the present invention is that the GC content herein is in a range of 40%-60%), and the existence of a poly T structure should be avoided. Finally, the *KLRrp6* gRNA-1 sequence determined by the present invention was CACCATGTCTTCAGAGGATA (SEQ ID NO.: 93), and *KLRrp6gRNA-2* sequence determined by the present invention was CCGACATGTTCAACAGAGTA (SEQ ID NO.: 94).

Method for construction and transformation of plasmids is as follows: two PCR amplification processes were carried out by using a pair of primers pCas9-KLRrp6-F1: ATGGGACTTTCACCATGTCTTCAGAGGATAGTTTTAGAGCTAGA AATAGCAAGTTAAAATAAGGCTAGTCC (SEQ ID NO.:59) and pCas9-KLRrp6-R1:
AGCTCTAAAACTATCCTCTGAAGACATGGTGAAAGTCCCATTCG CCACCCG (SEQ ID NO.:60) with a pCAS plasmid as template, and by using a pair of primers pCas9-KLRrp6-F2: ATGGGACTTTCCGACATGTTCAACAGAGTAGTTTTAGAGCTAGA AATAGCAAGTTAAAATAAGGCTAGTCC (SEQ ID NO.:61) and pCas9-KL Rrp6-R2: AGCTCTAAAACTACTCTGTTGAACATGTCGGAAAGTCCCATTCG CCACCCG (SEQ ID NO.:62) with a pCAS plasmid as template, respectively. 17 µL product of each PCR amplification process were mixed, followed by the addition of 1 µL of Dpn I and 2 µL 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1mL of an LB liquid medium, and then cultured with shaking at 37 °C for 1 hour. Thereafter, the mixture was coated onto a Kan-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named pHoCas9_SE_Kana_tRNA_ScRNR2_KLRrp6-1 and pHoCas9_SE_Kana_tRNA_ScRNR2_KLRrp6-2, respectively. One PCR amplification process was carried out by using primers of pCas9-F1: TAGGTCTAGAGATCTGTTTAGCTTGCCTCG (SEQ ID NO.:63) and pCas9-R1: TATCCACTAGACAGAAGTTTGCGTTCC (SEQ ID NO.:64) with pHoCas9_SE_Kana_tRNA_ScRNR2_KLRrp6-1 as template, and another PCR amplification process was carried out by using primers of pCas9-F2:
TATGGAACGCAAACTTCTGTCTAGTGGATAGTATATGTGTTATGT AGTATACTCTTTCTTCAACAATTAAATACTCTCGG (SEQ ID NO.:65) and pCas9-R2: CGAGGCAAGCTAAACAGATCTCTAGACCTATATCCACTAGACAG AAGTTTGCGTTCC (SEQ ID NO.:66) with pHoCas9_SE_Kana_tRNA_ScRNR2_KLRrp6-2 as template. PCR amplification products of pCas9-F1/pCas9-R1and pCas9-F2/pCas9-R2 were mixed at a ratio of 1:5, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated on a Kan-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named pHoCas9_SE_Kana_tRNA_ScRNR2_KLRrp6-1&2, respectively (as shown in Figure 16).

### 5.2 Construction and amplification of donor DNA plasmid

To the facilitate storage and amplification of linear donor DNA, the donor DNA was firstly inserted into a pMD18 plasmid, and then amplified by PCR to obtain a linear donor DNA sequence.

One PCR amplification process was carried out by using primers of KLRrp6-F1: CCCGGGGATCCTCTAGAGATGCGATAGCTTTAATCTGAGTGAAC ACCG (SEQ ID NO.:67) and KLRrp6-R1: CATGCCTGCAGGTCGACGATGGGTACTCGTTGATAACATGATGC GTAG (SEQ ID NO.:68) with a *Kluyveromyces lactis* genomic DNA as template; another PCR amplification process was carried out by using primers of pMD18-F: ATCGTCGACCTGCAGGCATG (SEQ ID NO.:69) and pMD18-R: ATCTCTAGAGGATCCCCGGG (SEQ ID NO.:70) with a pMD18 plasmid as template. Products of the two PCR amplification processes (8.5 µL for each PCR product) were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated on an Amp-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named KLRrp6-pMD18.

PCR amplification was carried out by using primers of KLRrp6-F2:
CTGACTCTAATCCACCAGCATCTTGAGCAGCTCTAATGGTATAAA TATCG (SEQ ID NO.:71) and KLRrp6-R2: GCTCAAGATGCTGGTGGATTAGAGTCAGCTGGTAGTCTAC (SEQ ID NO.:72) with a KLRrp6-pMD18 plasmid as template; KLRrp6-DD-pMD18 was constructed (as shown in Figure 17). Specific steps include as follows: the two PCR products (8.5 µL for each PCR product) were mixed, followed by the addition of 1 µL of Dpn I and 2 µL 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored.

### 5.3 Transformation and positive identification of Kluyveromyces lactis

5.3.1 A *Kluyveromyces lactis* solution was streaked and cultured on YPD solid medium. Monoclonal colonies were picked out, and then cultured with shaking overnight in 25 mL 2×YPD liquid medium. 2 mL of the yeast solution was taken and further cultured with shaking in 50 mL 2×YPD liquid medium for 2 to 8 hours. Subsequently, yeast cells were collected by centrifugation at 3,000 g for 5 minutes under the condition of 20 °C, and resuspended with the addition of 500 µL of sterile water. Similarly, cells were collected by centrifugation under the same condition. A competent cell solution (5% v/v glycerol and10% v/v DMSO) was prepared and yeast cells were dissolved in 500 µL of this solution. 50 µL of aliquots were aliquoted into 1.5 mL centrifuge tubes and stored at -80 °C.

5.3.2 Competent cells were placed at 37 °C for thawing for 15 to 30 seconds, centrifuged at 13,000 g for 2 minutes, and the supernatant was removed. Transformation buffer solution was prepared as follows: 260 µL of PEG 3350 (50% (w/v)), 36 µL of LiAc (1.0 M), 20 µL of carrier DNA (5.0 mg/mL), 15 µL of Cas9/gRNA plasmid and 10 µL of donor DNA were provided; then sterile water was added to form a mixture of a final volume of 360 µL. After heatshock, centrifugation was carried out at 13,000 g for 30 seconds to remove the supernatant. 1 mL of YPD liquid medium was added, and the mixture was incubated for 2 to 3 hours. 200 µL of the mixture was pipetted and coated onto an YPD (200 µg/mL G418) solid medium and then incubated for 2 to 3 days until monoclonal colonies grew out.

5.3.3 10 to 20 monoclonal colonies were picked out from a plate with transformed *Kluyveromyces lactis* cells. The monoclonal colonies were placed in 1 mL YPD (200 µg/mL G418) and cultured with shaking overnight. Samples were detected by PCR using CRISPR Insertion Check primers of K1Rrp6-CICF1: TGGAGGCGTACAATGCAGTG (SEQ ID NO.:73) and K1Rrp6-CICR1: TGAACCCTCT TCCATGTCTC ATC (SEQ ID NO.:74) with the yeast solution as template. Strains that were tested positive by PCR and identified by sequencing, were determined as positive strains.

### Comparative Example 6 Targeted Knock-out of NGL2 Gene via CRISPR-Cas9

### 6.1 Search for KlNGL2 sequence and determination of CRISPR gRNA sequence

### 6.1.1 Construction of cloning vector plasmid for targeted knock-out of gene

In KEGG database, BLAST alignment analysis was carried out based on *NGL3* gene. As a result, the gene sequence (SEQ ID NO.5) of *NGL* in *Kluyveromyces lactis* which has a code of K1LA0C06248g in the KEGG database was determined. The determined gene sequence has a 46.26% gene sequence homology and a 46.34% protein sequence homology to *NGL3* in *S. cerevisiae,* has a 45.42% gene sequence homology and a 29.07% protein sequence homology to *NGL2* in *S. pombe,* and has a 38.72% gene sequence homology and a 19.89% protein sequence homology to *NGL3* in *H. sapiens* (as shown in Figure 9). The determined gene sequence has a characteristic Exonuclease-Endonuclease-Phosphatase (EEP) domain (as shown in Figure 4). The gene is named *KlNGL2* (located at 552493...554043 of chromosome C).

The PAM sequence (NGG) was searched for nearby the initiation codon and the termination codon of the *KlNGL2* gene, and gRNA sequences were determined. The principle for selecting the gRNA is as follows: the GC content should be moderate (wherein, the standard in the present invention is that the GC content is in a range of 40%-60%), and the existence of a poly T structure should be avoided. Finally, the *KlNGL2* gRNA-1 sequence determined by the present invention was GCTGGTAGTACGCAAGACAC (SEQ ID NO.:75), and *KlNGL2* gRNA-2 sequence determined by the present invention was TTGTGCATGATTGTTAAACT (SEQ ID NO.:76).

Method for construction and transformation of plasmids is as follows: two PCR amplification processes were carried out by using a pair of primers pCas9-KLNGL2-F1: TATCCAGACACCAAAGTCAGGTTTTAGAGCTAGAAATAGCAAG TTAAAATAAGGCTAGTC (SEQ ID NO.:77) and pCas9-KLNGL3-R1: GCTCTAAAACCTGACTTTGGTGTCTGGATAAAAGTCCCATTCGC CACCCG (SEQ ID NO.:78) with a pCAS plasmid as template, and by using a pair of primers pCas9-KLNGL2-F2: TTGTGCATGATTGTTAAACTGTTTTAGAGCTAGAAATAGCAAGTT AAAATAAGGCTAGT (SEQ ID NO.:79) and pCas9-KLNGL2-R2: CGGGTGGCGAATGGGACTTTTTGTGCATGATTGTTAAACTGTTT TAGAGC (SEQ ID NO.:80) with a pCAS plasmid as template, respectively. 17 µL product of each PCR amplification process were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated on a Kan-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named pHoCas9_SE_Kana_tRNA_ScRNR2_KLNGL2-1 and pHoCas9_SE_Kana_tRNA_ScRNR2_KLNGL3-2, respectively. One PCR amplification process was carried out by using primers of pCas9-F1: TAGGTCTAGAGATCTGTTTAGCTTGCCTCG (SEQ ID NO.:81) and pCas9-R1: TATCCACTAGACAGAAGTTTGCGTTCC (SEQ ID NO.:82) with pHoCas9_SE_Kana_tRNA_ScRNR2_KLNGL2-1 as template. Another PCR amplification process was carried out by using primers pCas9-F2:
TATGGAACGCAAACTTCTGTCTAGTGGATAGTATATGTGTTATGT AGTATACTCTTTCTTCAACAATTAAATACTCTCGG (SEQ ID NO.:83) and pCas9-R2: CGAGGCAAGCTAAACAGATCTCTAGACCTATATCCACTAGACAG AAGTTTGCGTTCC (SEQ ID NO.:84) with pHoCas9_SE_Kana_tRNA_ScRNR2_KLNGL2-2 as template. PCR amplification products of pCas9-F1/pCas9-R1 and pCas9-F2/pCas9-R2 were mixed at a ratio of 1:5, followed by the addition of 1 µL of Dpn I and 2 µL of 10 × digestion buffer, and then the mixture was incubated at 37 °C for 3 hours. 10 µL of Dpn I-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated on a Kan-resistant LB solid medium, and then an inverted culture was carried out at 37 °C until monoclonal colonies grew out. Five monoclonal colonies were picked out and cultured with shaking in an LB liquid medium. After being detected PCR-positive and confirmed by sequencing, the plasmids were extracted and stored, and named pHoCas9_SE_Kana_tRNA_ScRNR2_KLNGL3-1&2, respectively (as shown in Figure 18).

### 6.2 Construction and amplification of donor DNA plasmid

To facilitate the storage and amplification of linear donor DNA, the donor DNA was firstly inserted into a pMD18 plasmid, and then amplified by PCR to obtain a linear donor DNA sequence.

One PCR amplification process was carried out by using primers of K1NGL2-F1: GAGCTCGGTACCCGGGGATCCTCTAGAGATCGAATACGTGAAA CAGCCTAGGAA (SEQ ID NO.:85) and K1NGL2-R1: GCCAAGCTTGCATGCCTGCAGGTCGACGATCACGGCCCTAGTA CTAATCCCAT (SEQ ID NO.:86) with a *Kluyveromyces lactis* genomic DNA as template; another PCR amplification process was carried out by using primers of pMD18-F: ATCGTCGACCTGCAGGCATG (SEQ ID NO.:87) and pMD18-R: ATCTCTAGAGGATCCCCGGG (SEQ ID NO.:88) with a pMD18 plasmid as template. Products of the two PCR amplification processes (8.5µL for each PCR product) were mixed, followed by the addition of 1 µL of Dpn I and 2µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at a 42 °C for 45 seconds, followed by the addition of 1 mL of LB liquid medium, and then cultured with shaking at 37 °C for 1 hour. Thereafter, the mixture was coated on an Amp-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored, and named K1NGL2-pMD18.

PCR amplification process was carried out by using primers of K1NGL2-F2:
GAAGTAATAATTTGAGCCAATATATTCATAAACTGTTTAACTATG GACTACACTACAG (SEQ ID NO.:89) and K1NGL2-R2: CCATAGTTAAACAGTTTATGAATATATTGGCTCAAATTATTACTTC TACTTTGCAGTG (SEQ ID NO.:90) with a KINGL3-pMD18 plasmid as template; K1NGL2-DD-pMD18 was constructed (as shown in Figure 19). Specific steps include as follows: the two PCR products (8.5 µL for each PCR product) were mixed, followed by the addition of 1 µL of Dpn I and 2 µL of 10 × digestion buffer, and then the mixture was incubated at 37 °C for 3 hours. 10 µL of DpnI-treated mixture was added into 100 µL of DH5α competent cells. The mixture was placed on ice for 30 minutes, heat-shocked at 42 °C for 45 seconds, followed by the addition of 1 mL of an LB liquid medium, and then cultured with shaking at 37 °C for 1 hour. Thereafter, the mixture was coated on an Amp-resistant LB solid medium, and then an inverted culture was carried out at 37 °C until monoclonal colonies grew out. Five monoclonal colonies were picked out and then cultured with shaking in an LB liquid medium. After being detected PCR-positive and confirmed by sequencing, the plasmids were extracted and stored.

### 6.3 Transformation and positive identification of Kluyveromyces lactis

6.3.1 A *Kluyveromyces lactis* solution was streaked and cultured on YPD solid medium. Monoclonal colonies were picked out, and cultured with shaking overnight in 25 mL 2×YPD liquid medium. 2 ml of the yeast solution was taken and further cultured with shaking in 50 mL 2×YPD liquid medium for 2 to 8 hours. Subsequently, yeast cells were collected by centrifugation at 3,000 g for 5 minutes under the condition of 20 °C, and resuspended with the addition of 500 µL of sterile water. Similarly, cells were collected by centrifugation under the same condition. A competent cell solution (5% v/v glycerol and10% v/v DMSO) was prepared and yeast cells were dissolved in 500 µL of this solution. 50 µL of aliquots were aliquoted into 1.5 mL centrifuge tubes and stored at -80 °C.

6.3.2 Competent cells were placed at 37 °C for thawing for f 15 to 30 seconds, centrifuged at 13,000 g for 2 minutes, and the supernatant was removed. Transformation buffer solution was prepared as follows: 260 µL of PEG 3350 (50% (w/v)), 36 µL of LiAc (1.0 M), 20 µL of carrier DNA (5.0 mg/mL), 15 µL of Cas9/gRNA plasmid, and 10 µL of donor DNA were added; then sterile water was added to form a mixture of a final volume of 360 µL. After heatshock, centrifugation was carried out at 13,000 g for 30 seconds to remove the supernatant. 1 mL of YPD liquid medium was added, and the mixture was incubated for 2 to 3 hours. 200 µL of the mixture was pipetted and coated onto an YPD (200 µg/mL G418) solid medium and then incubated for 2 to 3 days until monoclonal colonies grew out.

6.3.3 10 to 20 monoclonal colonies were picked out from a plate with transformed *Kluyveromyces lactis* cells. The monoclonal colonies were placed in 1 mL YPD (200 µg/mL G418) and cultured with shaking overnight. Samples were detected by PCR using CRISPR Insertion Check primers of K1NGL2-CICF1: ATATTGTCTAGCAGCTCATCGCGTA (SEQ ID NO.:91) and K1NGL2-CICR1: AAGCAGATTTATGCACGAATTGCC (SEQ ID NO.:92) with the yeast solution as template. Strains that were tested positive by PCR and identified by sequencing, were determined as positive strains.

### Example 7 Preparation of Cell-Free in vitro Protein Synthesis System and Assay of Protein Synthesis Efficiency

### Experimental method:

i. Preparation for the stored solution of the *in vitro* protein synthesis system: 1 M 4-hydroxyethyl piperazineethanesulfonic acid with pH of 7.4; 5 M potassium acetate; 250 mM magnesium acetate; 25 mM of a mixture of four nucleoside triphosphates, including ATP, GTP, CTP and UTP; 1 mM of a mixture of 20 types of amino acids, including: glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine, wherein, the concentration of each type of amino acid was 1.0 mM; 1 M phosphocreatine, 1 M dithiothreitol (DTT); 6.48 mg/mL creatine phosphokinase; 1.7 mg/mL T7 RNA polymerase; 20%-50% PEG 3350 or PEG 8000; and 20%-40% sucrose;
ii. the *in vitro* protein synthesis reaction system, comprising (in terms of final concentration): 22 mM of 4-hydroxyethyl piperazineethanesulfonic acid with pH of 7.4, 30-150 mM potassium acetate, 1.0-5.0 mM magnesium acetate, 1.5 mM - 4 mM of a mixture of four nucleoside triphosphates (including ATP, GTP, CTP and UTP), 0.08-0.24 mM amino acid mixture (including, glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine), 25 mM phosphocreatine, 1.7 mM dithiothreitol (DTT), 0.27 mg/ml creatine phosphokinase, 8-20 ng/µL DNA of firefly luciferase, 0.027-0.054 mg/mL T7 RNA polymerase, 1%-4% PEG, and finally added 50% by volume of yeast cell extract;
iii. *in vitro* protein synthesis reaction: the above-mentioned reaction system was placed in an environment of 20-30 °C, and let the mixture stand for a reaction for 2 to 6 hours;
iv. assay of the activity of luciferase: after completion of the reaction, an equal volume of luciferin as substrate was added into the wells for above reactions of a 96-well white plate or a 384-well white plate, which was immediately placed in an Envision 2120 multifunctional microplate reader (Perkin Elmer); and the absorbance was read to detect the activity of firefly luciferase, where the unit of activity is relative light unit (RLU) value, as shown in Figure 20.

### Experimental results:

The assay results of Example 7 indicate that among all mutant strains with nuclease being knocked out, the *ΔKLEXN53* strain can significantly enhance the efficiency of protein production in a yeast-based *in vitro* protein synthesis system (as shown in Table 2). It also indicates that the wild-type strain had a luciferase activity value of 2.90x10⁸ in an IVTT system, and the *ΔKLEXN53* yeast strain had a luciferase activity value of 7.16×10⁸ in the IVTT system, wherein, the luciferase activity value of *ΔKLEXN53* was 2.46-folds of that of the wild-type strain (as shown in Figure 20).

**Table 2**

| | Luciferase activity in IVTT (RLU) | Activity ratio of *ΔKLEXN53* strain to wild-type strain (RLU value of *ΔKLEXN53* strain /RLU value of wild-type strain) |
|---|---|---|
| *ΔKLEXN53* strain | 7.16×10⁸ | 2.46 folds |
| *ΔKLNGL2* strain | 5.83×10⁸ | 2.03 folds |

### References:

1. Ayyar, B.V., S. Arora, and R. O'Kennedy, Coming-of-Age of Antibodies in Cancer Therapeutics. Trends PharmacolSci, 2016. 37(12): p. 1009-1028.
2. Scott, A.M., J.D. Wolchok, and L.J., Antibody therapy of cancer. Nature Reviews Cancer, 2012. 12(4): p. 278.
3. Sonenberg, N. and A.G. Hinnebusch, Regulation of translation initiation in eukaryotes: mechanisms and biological targets. Cell, 2009. 136(4): p. 731-45.
4. M., D.J.R.G., Nucleic Acid. Encyclopedia of Cell Biology, Elsevier, 2015.
5. Chong, S., Overview of Cell-Free Protein Synthesis: Historic Landmarks, Commercial Systems, and Expanding Applications. 2014: John Wiley & Sons, Inc. 16.30.1-16.30.11.

All documents mentioned in the present invention are cited as references in this application, just as each document is individually cited as a reference.

## Claims

1. An *in vitro* cell-free protein synthesis system, comprising:
(a) yeast cell extract;
(b) polyethylene glycol;
(c) optional exogenous sucrose; and
(d) optional solvent, wherein the solvent is water or aqueous solvent,
wherein the yeast cell extract is derived from yeast cells, and expression or activity of *EXN53* gene in the yeast cells is reduced through CRISPR-Cas9 gene editing technology compared with that in wild-type yeast cells;
wherein the yeast cell extract comprises EXN53 protein, and content of the EXN53 protein in the yeast cell extract is equal to or less than 10%, preferably equal to or less than 5%, and more preferably equal to or less than 2%, based on the total weight of the yeast cell extract;
wherein amino acid sequence of the EXN53 protein is SEQ ID NO.:6.

2. The *in vitro* cell-free protein synthesis system of claim 1, wherein the EXN53 protein is derived from one or more sources of yeasts selected from the group consisting of *Pichia pastoris* and *Kluyveromyces,* and preferably derived from *Kluyveromyces.*

3. The *in vitro* cell-free protein synthesis system of claim 1, wherein nucleotide sequence of the EXN53 protein is SEQ ID NO.:1.

4. The *in vitro* cell-free protein synthesis system of claim 1, wherein the content of the EXN53 protein in the yeast cell extract is zero.

5. The *in vitro* cell-free protein synthesis system of claim 1, wherein, the cell-free protein synthesis system further comprises one or more components selected from the group consisting of:
(e1) substrate for synthesizing RNA;
(e2) substrate for synthesizing protein;
(e3) magnesium ion;
(e4) potassium ion;
(e5) buffer solvent;
(e6) RNA polymerase; and
(e7) energy regeneration system.

6. A method for producing the *in vitro* cell-free protein synthesis system of claim 1, comprising a step of:
mixing components of (a) the yeast cell extract, (b) polyethylene glycol, (c) optional exogenous sucrose, and (d) the optional solvent, to obtain the *in vitro* cell-free protein synthesis system of claim 1, wherein the solvent is water or aqueous solvent; the yeast cell extract contains the EXN53 protein, and the content of the EXN53 protein in the yeast cell extract is equal to or less than 10%, preferably equal to or less than 5%, and more preferably equal to or less than 2%.

7. A method for *in vitro* protein synthesis, comprising steps of:
(i) providing the *in vitro* cell-free protein synthesis system of claim 1, and adding exogenous DNA molecules for guiding protein synthesis, wherein the content of the EXN53 protein in the protein synthesis system is equal to or less than 10%, preferably equal to or less than 5%, and more preferably equal to or less than 2%; and
(ii) incubating the *in vitro* cell-free protein synthesis system provided in the step (i) for a period of time T1 under suitable conditions to synthesize protein encoded by the exogenous DNA.

8. The method for *in vitro* protein synthesis of claim 7, wherein the method further comprises: (iii) isolating the protein encoded by the exogenous DNA from the protein synthesis system;
detecting the protein encoded by the exogenous DNA from the protein synthesis system; or
their combination.

9. The method for *in vitro* protein synthesis of claim 7, wherein the protein encoded by the exogenous DNA is selected from the group consisting of luciferin, luciferase, green fluorescent protein, yellow fluorescent protein, aminoacyl tRNA synthetase, glyceraldehyde-3-phosphate dehydrogenase, catalase, actin, variable regions of antibodies, luciferase mutants, α-amylase, enterocin A, Hepatitis C virus E2 glycoprotein, insulin precursors, interferon αA, interleukin-1β, lysozyme, serum albumins, single-chain variable fragment of antibodies, transthyretin, tyrosinase, xylanase, and any combination thereof.

10. An engineering strain, wherein the engineering strain is a *Kluyveromyces* strain, and
expression level or activity of *EXN53* gene in the *Kluyveromyces* strain is reduced through CRISPR-Cas9 gene editing technology compared with that in wild-type *Kluyveromyces* strain; wherein, the *EXN53* gene is a nuclease gene;
wherein amino acid sequence of the EXN53 protein is SEQ ID NO.:6.

11. The engineering strain of claim 10, wherein the reduction of the expression level or activity corresponds to that the expression level of the *EXN53* gene is equal to or less than 10%, preferably equal to or less than 5%, and more preferably equal to or less than 2%.

12. The engineering strain of claim 10, wherein the reduction of the expression level or activity of the *EXN53* gene satisfies the following condition:
the ratio of A1 to A0 is equal to or less than 30%, preferably equal to or less than 10%, more preferably equal to or less than 5%, more preferably equal to or less than 2%, and most preferably in a range of 0 to 2%;
wherein, A1 corresponds to the expression level of the *EXN53* gene, and A0 corresponds to the expression level of wild-type *EXN53* gene; or
A1 corresponds to the activity of the *EXN53* gene, and A0 corresponds to the activity of the wild-type *EXN53* gene.

13. A use of the engineering strain of claim 10, for improving the efficiency of *in vitro* protein synthesis.

## Patentansprüche

1. - Zellfreies in-vitro-Proteinsynthesesystem, umfassend:
(a) Hefezellenextrakt;
(b) Polyethylenglykol;
(c) optional exogene Saccharose; und
(d) optional Lösungsmittel, wobei das Lösungsmittel Wasser oder wässriges Lösungsmittel ist,
wobei der Hefezellenextrakt aus Hefezellen abgeleitet ist und eine Expression oder Aktivität von *EXN53-Gen* in den Hefezellen durch CRISPR-Cas9-Geneditierungstechnologie im Vergleich zu jener in Wildtyp-Hefezellen reduziert ist;
wobei der Hefezellenextrakt EXN53-Protein umfasst und der Gehalt des EXN53-Proteins in dem Hefezellenextrakt gleich wie oder weniger als 10 %, vorzugsweise gleich wie oder weniger als 5 % und bevorzugter gleich wie oder weniger als 2 %, bezogen auf das Gesamtgewicht des Hefezellenextrakts, ist;
wobei Aminosäuresequenz des EXN53-Proteins SEQ ID NO: 6 ist.

2. - Zellfreies *in-vitro*-Proteinsynthesesystem nach Anspruch 1, wobei das EXN53-Protein aus einer oder mehreren Quellen von Hefen, ausgewählt aus der Gruppe, bestehend aus *Pichia pastoris* und *Kluyveromyces,* und vorzugsweise aus *Kluyveromyces,* abgeleitet ist.

3. - Zellfreies *in-vitro*-Proteinsynthesesystem nach Anspruch 1, wobei die Nukleotidsequenz des EXN53-Proteins SEQ ID NO: 1 ist.

4. - Zellfreies *in-vitro*-Proteinsynthesesystem nach Anspruch 1, wobei der Gehalt des EXN53-Proteins in dem Hefezellenextrakt gleich null ist.

5. - Zellfreies *in-vitro*-Proteinsynthesesystem nach Anspruch 1, wobei das zellfreie Proteinsynthesesystem ferner eine oder mehrere Komponenten umfasst, die ausgewählt sind aus der Gruppe, bestehend aus:
(e1) Substrat zur Synthese von RNA;
(e2) Substrat zur Synthese von Protein;
(e3) Magnesiumion;
(e4) Kaliumion;
(e5) Puffer-Lösungsmittel;
(e6) RNA-Polymerase; und
(e7) Energierückgewinnungssystem.

6. - Verfahren zum Herstellen des zellfreien *In-vitro*-Proteinsynthesesystems nach Anspruch 1, umfassend einen folgenden Schritt:
Mischen von Komponenten von (a) dem Hefezellextrakt, (b) Polyethylenglykol, (c) optionaler exogener Saccharose und (d) dem optionalen Lösungsmittel, um das zellfreie *in-vitro-*Proteinsynthesesystem nach Anspruch 1 zu erlangen, wobei das Lösungsmittel Wasser oder wässriges Lösungsmittel ist; der Hefezellextrakt das EXN53-Protein enthält und der Gehalt des EXN53-Proteins in dem Hefezellextrakt gleich wie oder weniger als 10 %, vorzugsweise gleich wie oder weniger als 5 % und bevorzugter gleich wie oder weniger als 2 % ist.

7. - Verfahren zur *in-vitro-Proteinsynthese,* umfassend die folgenden Schritte:
(i) Bereitstellen des zellfreien *in-vitro-*Proteinsynthesesystems nach Anspruch 1 und Hinzufügen exogener DNA-Moleküle zum Steuern von Proteinsynthese, wobei der Gehalt des EXN53-Proteins im Proteinsynthesesystem gleich wie oder weniger als 10 %, vorzugsweise gleich wie oder weniger als 5 % und noch bevorzugter gleich wie oder weniger als 2 % ist; und
(ii) Inkubieren des zellfreien *in-vitro-*Proteinsynthesesystems, das in Schritt (i) bereitgestellt wird, für eine Zeitspanne T1 unter geeigneten Bedingungen, um das von der exogenen DNA kodierte Protein zu synthetisieren.

8. - Zellfreies *in-vitro*-Proteinsynthesesystem nach Anspruch 7, wobei das Verfahren ferner Folgendes umfasst: (iii) Isolieren des von der exogenen DNA kodierten Proteins aus dem Proteinsynthesesystem;
Detektieren des von der exogenen DNA kodierten Proteins aus dem Proteinsynthesesystem; oder
dessen Kombination.

9. - Zellfreies *in-vitro-*Proteinsynthesesystem nach Anspruch 7, wobei das von der exogenen DNA kodierte Protein ausgewählt ist aus der Gruppe, bestehend aus Luciferin, Luciferase, grün fluoreszierendem Protein, gelb fluoreszierendem Protein, Aminoacyl-tRNA-Synthetase, Glyceraldehyd-3-phosphat-Dehydrogenase, Katalase, Actin, variablen Bereichen von Antikörpern, Luciferase-Mutanten, α-Amylase, Enterocin A, Hepatitis-C-Virus-E2-Glykoprotein, Insulinvorläufern, Interferon αA, Interleukin-1ß, Lysozym, Serumalbuminen, einkettigem variablem Fragment von Antikörpern, Transthyretin, Tyrosinase, Xylanase und einer beliebigen Kombination davon.

10. - Technischer Stamm, wobei der technische Stamm ein *Kluyveromyces-*Stamm ist, und
ein Expressionsniveau oder Aktivität des *EXN53-Gens* in dem *Kluyveromyces-*Stamm durch CRISPR-Cas9-Geneditierungstechnologie im Vergleich zu dem Wildtyp-*Kuyveromyces-*Stamm reduziert ist; wobei das *EXN53-Gen* ein Nuklease-Gen ist;
wobei die Aminosäuresequenz des EXN53-Proteins SEQ ID NO: 6 ist.

11. - Modifizierender Stamm nach Anspruch 10, wobei die Verringerung des Expressionsniveaus oder der Aktivität jenem entspricht, dass das Expressionsniveau des *EXN53-Gens* gleich wie oder weniger als 10 %, vorzugsweise gleich wie oder weniger als 5 % und bevorzugter gleich wie oder weniger als 2 % ist.

12. - Modifizierender Stamm nach Anspruch 10, wobei die Verringerung des Expressionsniveaus oder der Aktivität des *EXN53-Gens* die folgende Bedingung erfüllt:
das Verhältnis von A1 zu A0 gleich wie oder weniger als 30 %, vorzugsweise gleich wie oder weniger als 10 %, bevorzugter gleich wie oder weniger als 5 %, bevorzugter gleich wie oder weniger als 2 % und am meisten bevorzugt in einem Bereich von 0 bis 2 % ist;
wobei A1 dem Expressionsniveau des *EXN53-Gens* entspricht und A0 dem Expressionsniveau des Wildtyp-*EXN53-*Gens entspricht; oder
A1 der Aktivität des *EXN53-Gens* entspricht und A0 der Aktivität des Wildtyp-*EXN53*-Gens entspricht.

13. - Verwendung des modifizierenden Stamms nach Anspruch 10 zum Verbessern der Effizienz von *in-vitro-*Proteinsynthese.

## Revendications

1. - Système *in vitro* de synthèse protéique acellulaire, comprenant :
(a) un extrait de cellules de levure ;
(b) du polyéthylène glycol ;
(c) du saccharose exogène facultatif ; et
(d) un solvant facultatif, le solvant étant de l'eau ou un solvant aqueux,
dans lequel l'extrait de cellules de levure est dérivé de cellules de levure, et l'expression ou l'activité du gène *EXN53* dans les cellules de levure est réduite par la technologie d'édition de gènes CRISPR-Cas9 par comparaison avec celle des cellules de levure de type sauvage ;
dans lequel l'extrait de cellules de levure comprend la protéine EXN53, et la teneur en protéine EXN53 dans l'extrait de cellules de levure est égale ou inférieure à 10 %, de préférence égale ou inférieure à 5 %, et de façon davantage préférée égale ou inférieure à 2 %, sur la base du poids total de l'extrait de cellules de levure ;
dans laquelle la séquence d'acides aminés de la protéine EXN53 est SED ID NO:6.

2. - Système de synthèse protéique acellulaire *in vitro* selon la revendication 1, dans lequel la protéine EXN53 est dérivée d'une ou plusieurs sources de levures choisies dans le groupe constitué par *Pichia pastoris* et *Kluyveromyces,* et de préférence dérivée de *Kluyveromyces.*

3. - Système de synthèse protéique acellulaire *in vitro* selon la revendication 1, dans lequel la séquence nucléotidique de la protéine EXN53 est SEQ ID NO:1.

4. - Système de synthèse protéique acellulaire *in vitro* selon la revendication 1, dans lequel la teneur en protéine EXN53 dans l'extrait de cellules de levure est nulle.

5. - Système de synthèse protéique acellulaire *in vitro* selon la revendication 1, dans lequel le système de synthèse protéique acellulaire comprend en outre un ou plusieurs composants choisis dans le groupe constitué par :
(e1) un substrat pour la synthèse de l'ARN ;
(e2) un substrat pour la synthèse de protéines ;
(e3) l'ion magnésium ;
(e4) l'ion potassium ;
(e5) un solvant tampon ;
(e6) l'ARN polymérase ; et
(e7) un système de régénération d'énergie.

6. - Procédé de production du système de synthèse protéique acellulaire *in vitro selon* la revendication 1, comprenant une étape consistant à :
mélanger les composants de (a) l'extrait de cellules de levure, (b) du polyéthylène glycol, (c) du saccharose exogène facultatif, et (d) le solvant facultatif, pour obtenir le système de synthèse protéique acellulaire *in vitro* de la revendication 1, le solvant étant de l'eau ou un solvant aqueux ; l'extrait de cellules de levure contenant la protéine EXN53, et la teneur de la protéine EXN53 dans l'extrait de cellules de levure est égale ou inférieure à 10 %, de préférence égale ou inférieure à 5 %, et de façon davantage préférée égale ou inférieure à 2 %.

7. - Procédé de synthèse protéique *in vitro,* comprenant les étapes consistant à :
(i) fournir le système de synthèse protéique acellulaire *in vitro* de la revendication 1, et ajouter des molécules d'ADN exogènes pour guider la synthèse protéique, la teneur en la protéine EXN53 dans le système de synthèse protéique étant égale ou inférieure à 10 %, de préférence égale ou inférieure à 5 %, et de façon davantage préférée égale ou inférieure à 2 % ; et
(ii) faire incuber le système de synthèse protéique acellulaire *in vitro* fourni à l'étape (i) pendant une période de temps T1 dans des conditions appropriées pour synthétiser la protéine codée par l'ADN exogène.

8. - Procédé de synthèse protéique *in vitro* selon la revendication 7, dans lequel le procédé comprend en outre : (iii) isoler la protéine codée par l'ADN exogène à partir du système de synthèse protéique ;
détecter la protéine codée par l'ADN exogène à partir du système de synthèse des protéines ; ou
leur combinaison.

9. - Procédé de synthèse protéique *in vitro* selon la revendication 7, dans lequel la protéine codée par l'ADN exogène est choisie dans le groupe constitué par la luciférine, la luciférase, la protéine fluorescente verte, la protéine fluorescente jaune, l'aminoacyl-ARNt synthétase, la glycéraldéhyde-3-phosphate déshydrogénase, la catalase, l'actine, les régions variables d'anticorps, les mutants de luciférase, l'a-amylase, l'entérocine A, la glycoprotéine E2 du virus de l'hépatite C, les précurseurs de l'insuline, l'interféron αA, l'interleukine-1β, le lysozyme, les sérum albumines, un fragment variable à chaîne unique d'anticorps, la transthyrétine, la tyrosinase, la xylanase, et toute combinaison de ceux-ci.

10. - Souche d'ingénierie génétique, dans laquelle la souche d'ingénierie génétique est une souche de *Kluyveromyces,* et
le niveau d'expression ou l'activité du gène *EXN53* dans la souche *Kluyveromyces* est réduit par la technologie d'édition de gènes CRISPR-Cas9 par comparaison avec celui de la souche *Kluyveromyces* de type sauvage, le gène *EXN53* étant un gène de nucléase ;
dans laquelle la séquence d'acides aminés de la protéine EXN53 est SED ID NO:6.

11. - Souche d'ingénierie génétique selon la revendication 10, dans laquelle la réduction du niveau d'expression ou de l'activité correspond à ce que le niveau d'expression du gène EXN53 soit égal ou inférieur à 10%, de préférence égal ou inférieur à 5%, et de façon davantage préférée égal ou inférieur à 2%.

12. - Souche d'ingénierie génétique selon la revendication 10, dans laquelle la réduction du niveau d'expression ou de l'activité du gène EXN53 répond à la condition suivante :
le rapport de A1 sur A0 est égal ou inférieur à 30 %, de préférence égal ou inférieur à 10 %, de façon davantage préférée égal ou inférieur à 5 %, de façon davantage préférée égal ou inférieur à 2 %, et de la façon que l'on préfère le plus dans une plage de 0 à 2 % ;
où A1 correspond au niveau d'expression du gène *EXN53,* et A0 correspond au niveau d'expression du gène *EXN53* de type sauvage ; ou
A1 correspond à l'activité du gène *EXN53,* et A0 correspond à l'activité du gène *EXN53* de type sauvage.

13. - Utilisation de la souche d'ingénierie génétique selon la revendication 10, pour améliorer l'efficacité de la synthèse protéique *in vitro.*
